# EUROPÄISCHE PATENTSCHRIFT

(11) **EP 3 273 900 B1**
(45) Veröffentlichungstag und Bekanntmachung des Hinweises auf die Patenterteilung: **08.05.2019**
(21) Anmeldenummer: 16712836.2
(22) Anmeldetag: 24.03.2016
(51) Int. Cl.: A61B 90/90, A61B 90/00, A61B 90/50

(54) **HALTEARM FÜR MEDIZINISCHE ZWECKE MIT ABNEHMBARER BEDIENEINRICHTUNG, SOWIE BEDIENEINRICHTUNG HIERFÜR**
HOLDING ARM FOR MEDICAL PURPOSES WITH DETACHABLE COMMAND UNIT AND COMMAND UNIT THEREFOR
BRAS DE RETENUE À USAGE MÉDICAL ÉQUIPÉ D'UN DISPOSITIF DE COMMANDE AMOVIBLE, ET DISPOSITIF DE COMMANDE DE CELUI-CI

(30) Priorität: 27.03.2015 DE 102015104819
(43) Veröffentlichungstag der Anmeldung: 31.01.2018
(73) Patentinhaber: medineering GmbH, 81829 München (DE)
(72) Erfinder: NOWATSCHIN, Stephan, 81677 München (DE); KRINNINGER, Maximilian, 82234 Weßling-Oberpfaffenhofen (DE); GIERLACH, Dominikus, 80798 München (DE)
(74) Vertreter: Eisenführ Speiser
(86) Internationale Anmeldenummer: PCT/EP2016/056570
(87) Internationale Veröffentlichungsnummer: WO 2016/156210

(56) Entgegenhaltungen:
- EP-A2- 1 520 548
- EP-B1- 1 958 587
- WO-A1-2014/139018
- DE-A1-102010 027 248
- US-A1- 2004 148 058

## Beschreibung

Die Erfindung betrifft einen Haltearm für medizinische Zwecke, insbesondere zu Halten eines chirurgischen mechatronischen Assistenzsystems und/oder eines chirurgischen Instruments, mit einem proximalen Ende zum Befestigen des Halterarms an einer Basis und einem distalen Ende zum Aufnehmen eines chirurgischen mechatronischen Assistenzsystems und/oder chirurgischen Instruments, wenigstens einem ersten und einem zweiten Armsegment, wobei das erste Armsegment mit einem ersten Gelenk und das zweite Armsegment mit einem zweiten Gelenk verbunden ist, wobei jedes Gelenk mittels einer Freigabeeinrichtung freigebbar und arretierbar ist, einer Bedieneinheit zum Verbringen des Haltearms an die gewünschte Pose, und einer Steuereinheit, die mit der Bedieneinheit und der Freigabeeinrichtung zum Übertragen von Signalen von der Bedieneinheit an die Freigabeeinrichtung gekoppelt ist. Ferner betrifft die Erfindung eine Bedieneinrichtung zur Verwendung mit dem Haltearm.

Haltearme der eingangs genannten Art sind bereits seit längerem bekannt und werden in der Chirurgie insbesondere dazu eingesetzt, einen Operateur von statischer Haltearbeit zu entlasten. Ein derartiger Haltearm wird eingesetzt, um ein mechatronisches Assistenzsystem und/oder ein chirurgisches Instrument zu halten, wie etwa einen Manipulator, ein Endoskop, eine chirurgische Klemme und dergleichen. Insbesondere zum Halten von

Endoskopen haben sich die eingangs genannten Haltearme bewährt. Bei der endoskopischen Chirurgie bedient ein Operateur in der Regel mit beiden Händen ein Instrument, während ein Assistent das Endoskop hält, um das Operationsfeld über einen Bildschirm sichtbar zu machen. Das Halten des Endoskops über einen längeren Zeitraum, ist sehr ermüdend. Aus diesem Grund werden vermehrt Haltearme eingesetzt.

Eine solcher Haltearm ist beispielsweise aus DE 195 26 915 B4 bekannt. Die dort offenbarte Haltevorrichtung für medizinische Zwecke weist ein Anschlussteil und einen Halter für chirurgische Werkzeuge sowie einen zwischen dem Halter und dem Anschlussteil angeordneten Arm auf. Der Arm ist mit dem Halter und dem Anschlussteil oder mit einem benachbarten Arm über ein Gelenk verbunden und mit einer pneumatisch betätigbaren Vorrichtung zur wahlweisen Festlegung und Lösung der Gelenke gekoppelt, wobei diese Vorrichtung die Gelenke unter Einwirkung einer eine Bremskraft auf das Gelenk ausübenden mechanischen Feder festlegt und wobei die Vorrichtung gegen die Kraft dieser Feder pneumatisch in einen das Gelenk freigebenden Zustand überführbar ist. An dem Halter am proximalen Ende des Arms ist ein Betätigungsorgan angeordnet, mittels dessen Hilfe ein Ventil öffenbar ist, sodass die einzelnen Gelenke des Arms verstellt werden können. Bei Loslassen des Betätigungsorgans wird das Ventil wieder geschlossen, sodass die Gelenke festgelegt sind.

Ein ähnlicher Haltearm ist in EP 1 958 587 B1 offenbart. Der dort offenbarte Haltearm weist ebenfalls mehrere Gelenke auf, und zur Betätigung der Gelenke ist ein berührungssensitiver Sensor vorgesehen. Dieser Sensor ist am Haltearm benachbart zum medizinischen Instrument angeordnet, sodass bei Ergreifen des medizinischen Instruments der Operator in Kontakt mit dem berührungssensitiven Sensor kommt, wodurch die Gelenke des Haltearms freigegeben werden.

Sowohl der in DE 195 26 915 B4 als auch in EP 1 958 587 B1 offenbarte Haltearm dient in erster Linie als eine Art Exoskelett für den Operator, sodass sich der Operateur bei der Operation auf diesem Haltearm abstützen kann und bei Ergreifen des medizinischen Instruments oder bei Betätigung des Betätigungsorgans alle Gelenke freigegeben werden, sodass die Pose des Haltearms verändert werden kann.

Aus DE 10 2004 050 714 A1 ist ein weiterer Haltearm bekannt, der zum Halten eines Endoskops ausgebildet ist. Der Arm weist eine Mehrzahl an Gelenken auf, die pneumatisch geschlossen werden können. Der Haltearm ist mit einem Fußschaltventil verbunden.

Bei Betätigung des Fußschaltventils gelangt Druckluft in sämtliche Gelenke, wodurch diese freigegeben werden.

Nachteilig ist allerdings, dass eine genaue Positionierung des an dem Haltearm angeordneten mechatronischen Assistenzsystems und/oder chirurgischen Instruments mit den vorbekannten Haltearmen nur schwer möglich ist und stark von dem Geschick des Operateurs abhängig ist. Die Positionsgenauigkeit ist allein auf die Fähigkeiten des Operateurs beschränkt, der das distale Ende des Arms im Raum positioniert.

Ein weiteres Problem besteht darin, dass die oben genannten Haltearme entweder universell oder für eine spezifische Anwendung ausgelegt sind. Es gibt beispielsweise Haltearme, die allgemein einsetzbar sind, unabhängig von der Operationsanwendung, und Halterarme, die spezifisch für ein Anwendungsfeld beispielsweise der Kopfchirurgie konzipiert sind. Bei Erstgenannten besteht das Problem, dass diesen teilweise Spezialfunktionen für spezialisierte Anwendungen fehlen, wohingegen die zweitgenannten nicht oder nur schlecht für andere Operationsfelder eingesetzt werden können.

Aus DE 10 2010 027 248 A1, welche den Oberbegriff von Anspruch 1 offenbart, ist eine Haltevorrichtung für ein Instrument bekannt, die wenigstens ein Gelenk mit wenigstens zwei relativ zueinander beweglichen Teilen und wenigstens eine dem Gelenk zugeordnete Antriebseinheit aufweist. Die Antriebseinheit ist dazu ausgebildet, das Gelenk zu bewegen, indem sie wenigstens eines der zueinander beweglichen Teile des Gelenks antreibt. Die Haltevorrichtung weist ferner eine Sicherungseinrichtung mit einer dem Gelenk zugeordneten Arretierung und mit einer aktivierbaren Freigabeeinheit auf, wobei die Arretierung derart mit der Freigabeeinheit zusammenwirkt, dass sie das Gelenk in einer Arretierposition hält, solange die Freigabeeinheit nicht aktiviert ist. Die Sicherungseinrichtung ist dazu eingerichtet, eine Bewegung des Gelenks innerhalb eines vorgegebenen Bewegungsbereichs aus der jeweiligen Arretierposition heraus auch dann zuzulassen, wenn die Freigabeeinheit nicht aktiviert ist.

Ferner offenbart WO 2014/139018 A1 einen Chirurgieroboter, dessen Arm ein optisches Aufnahmegerät zur Bildwiedergabe des Operationsbereiches trägt und der in Abhängigkeit eines, innerhalb des Operationsbereichs delektierten Instruments seine Betriebsparameter zwar dementsprechend automatisch anpasst, so dass eine optimale Bildwiedergabe des chirurgischen Vorgangs erfolgt.

Aufgabe der vorliegenden Erfindung ist es einen Haltearm sowie eine Bedieneinrichtung der eingangs genannten Art anzugeben, die auf einfache und sichere Weise an einen jeweiligen Operationsfall anpassbar sind.

Diese Aufgabe wird bei der Haltearm eingangs genannten Art mit den Merkmalen des Anspruchs 1 gelöst, also insbesondere durch eine Schnittstelle zur Aufnahme eines Schlüssels, und einem Erfassungsmittel zum Erfassen einer Identität des Schlüssels, wobei die Steuereinheit dazu eingerichtet ist, die Freigabeeinrichtung in Abhängigkeit von der Identität des aufgenommenen Schlüssels zu steuern.

Indem die Steuereinheit dazu eingerichtet ist die Freigabeeinrichtung in Abhängigkeit von der Identität des aufgenommenen Schlüssels zu steuern, und vorzugsweise die Freigabeeinrichtung in Anhängigkeit von der Identität des aufgenommenen Schlüssels steuert, ist der Haltearm insgesamt, durch Auswahl eines entsprechenden Schlüssels mit entsprechender Identität an die speziellen Bedingungen einer Operation aus einem bestimmten Operationsbereich anpassbar. Die Freigabeeinrichtung weist vorzugsweise eine aktive Bremse in jedem Gelenk auf. Alternativ oder zusätzlich weist die Freigabeeinrichtung einen Antrieb in jedem Gelenk des Haltearms auf. Die Steuereinheit ist vorzugsweise mit den Erfassungsmitteln gekoppelt, um die erfasste Identität zu empfangen. Dies kann über ein Bussystem im Inneren des Haltearms realisiert werden.

Der Haltearm weist eine Bedieneinheit auf, die zum Verbringen des Haltearms in eine gewünschte Pose ausgebildet ist, wobei die Bedieneinheit dazu eingerichtet ist, bei Kontakt zwischen einer Bedienperson und einem der ersten und zweiten Armsegmente, insbesondere daran angeordneten Bedieneinrichtungen, das zugehörige Gelenk freizugeben. Es ist daher bevorzugt vorgesehen, dass die Bedieneinheit dazu eingerichtet ist, bei Kontakt zwischen einer Bedienperson und dem ersten Armsegment das erste Gelenk freizugeben und bei Kontakt zwischen einer Bedienperson und dem zweiten Armsegment das zweite Gelenk freizugeben. Bei Kontakt zwischen einer Bedienperson mit einem entsprechenden Armsegment wird vorzugsweise nur das zugeordnete Gelenk freigegeben. Dadurch ist es möglich, intuitiv einzelne Gelenke zu bewegen und den Haltearm so segmentweise zu verstellen und in eine gewünschte Pose zu bringen. Dadurch ist eine genauere Positionierung möglich, da inkrementell jedes Segment separat verstellt werden kann. Es ist ebenso möglich, mehrere Segmente auf einmal zu kontaktieren, sodass mehrere Gelenke gleichzeitig freigegeben werden und so verstellbar sind. Dadurch ist es möglich, den Haltearm auf einfache Art und Weise, insbesondere intuitiv, in eine gewünschte Pose zu überführen.

Neben den ersten und zweiten Armsegmenten sind vorzugsweise weitere Armsegmente vorgesehen, die ebenfalls jeweils einem Gelenk zugeordnet sind. Die Armsegmente selbst sind im Wesentlichen starr und vorzugsweise im Wesentlichen stabförmig. Der Begriff "stabförmig" umfasst hier sowohl im Wesentlichen gerade Armsegmente als auch leicht bis stark gekrümmte Armsegmente. Bei einem derartigen Haltearm wechseln sich Armsegmente und Gelenke stets ab, wobei der Haltearm am distalen und am proximalen Ende sowohl mit einem Gelenk als auch mit einem Segment oder einem Anschlusselement enden kann. Mit dem proximalen Ende ist der Haltearm an einer Basis befestigbar. Die Basis kann alternativ fest mit dem Arm gekoppelt sein, oder der Arm ist von der Basis entnehmbar. Die Basis ist in einer Ausführungsform als Operationstisch ausgebildet, und der Haltearm ist mit einem Operationstisch koppelbar. Vorzugsweise ist der Haltearm mit einer an dem Operationstisch vorgesehenen Normschiene koppelbar. Derartige Normschienen sind in der Regel bei Operationstischen vorgesehen, sodass an dem Haltearm zur Kopplung mit der Normschiene eines Operationstisches eine Standardschnittstelle vorgesehen sein kann. Übliche Operationstische sind zudem aus einzelnen Segmenten zusammengesetzt. Zur Kopplung weisen sie Segmente an den Stirnseiten entsprechende Koppelstellen auf, die in der Regel herstellerspezifisch sind. Bevorzugt ist der Haltearm über eine solche Koppelstelle an dem Operationstisch befestigbar. Dazu kann an dem proximalen Ende ein herstellerspezifischer Adapter vorgesehen sein. Alternativ ist die Basis als eine separate Vorrichtung, wie etwa ein Ständer, der auf dem Boden eines Operationssaales aufstellbar ist. In einer weiteren Alternative ist die Basis als eine Halterung ausgebildet, die beispielsweise an einer Wand oder Decke eines Operationssaales befestigbar ist.

Der Haltearm ist vorzugsweise als sogenannter passiver Haltearm ausgebildet und weist daher ausschließlich aktiv gebremste Gelenke auf, jedoch keine angetriebenen Gelenke, wie dies bei robotischen Haltearmen häufig der Fall ist. Jedes Gelenk ist daher nur freigeb- und arretierbar, allerdings nicht antreibbar. Hierdurch ist der Haltearm einfach aufgebaut und bedarf keiner komplexen Steuerung zu seinem Betrieb.

Vorzugsweise weist der Haltearm eine erste Schnittstelle an dem proximalen Ende zum Verbinden des Haltearms mit einer Energiequelle sowie mit einer externen Steuereinheit zum Übertragen von Signalen an den und von dem Haltearm; eine zweite Schnittstelle an dem distalen Ende zum Koppeln des Haltearms mit dem Assistenzsystem zum Steuern des Assistenzsystems; und eine Übertragungseinrichtung, welche innerhalb des Haltearms angeordnet ist und die erste Schnittstelle mit der zweiten Schnittstelle zum Übertragen von Energie und Signalen zwischen den Schnittstellen verbindet. Die Schnittstellen und auch ein internes Versorgungssystem und/oder Daten-Bussystem ist vorzugsweise wie in DE 10 2014 016 824 der hiesigen Anmelderin ausgebildet.

Als Assistenzsysteme in dem erfindungsgemäßen Sinn werden jegliche Art von mechatronischen Manipulatoren, die in der Chirurgie eingesetzt werden, verstanden, so wie insbesondere Endoskope, Exoskope, Laparoskope, Trokare und dergleichen. Die zweite Schnittstelle am distalen Ende des Haltearms ist dazu ausgebildet, sowohl mechanisch mit dem Assistenzsystem zu koppeln, um dieses in einer definierten Position zum Haltearm zu halten, als auch die notwendigen weiteren Anschlüsse, wie insbesondere einen Anschluss für elektrische Energie und einen Anschluss zum Übertragen von Signalen, insbesondere Stellsignalen, bereitzustellen. Innerhalb des Haltearms ist eine Übertragungseinrichtung ausgebildet, die vorzugsweise ein Bussystem aufweist. Ferner weist die Übertragungseinrichtung Mittel zum Übertragen von elektrischer Energie auf. Dadurch sind sämtliche Kabel, die zur Übertragung von elektrischer Energie und/oder Daten von der ersten Schnittstelle zur zweiten Schnittstelle erforderlich sind, im Inneren des Haltearms angeordnet und dadurch während des Betriebs des Haltearms geschützt. An der ersten Schnittstelle sind weiterhin Mittel vorgesehen, um den Haltearm mit einer Energiequelle und einer externen Steuereinheit, wie etwa einem Computer und/oder einem OP-System, zu koppeln. Hierdurch ist der Einsatzbereich des Haltearms vergrößert, und dieser kann flexibel für verschiedene Assistenzsysteme eingesetzt werden. Gleichzeitig ist die Sicherheit verbessert, da das Anbringen zusätzlicher Kabel oder dergleichen nicht erforderlich ist, sondern das Assistenzsystem ist lediglich mit der zweiten Schnittstelle am distalen Ende zu koppeln und der Haltearm selbst wiederum über die erste Schnittstelle am proximalen Ende mit einer Energiequelle und einer externen Steuereinheit koppelbar.

In einer ersten Bevorzugten Ausführungsform weist die Bedieneinheit die Schnittstelle zur Aufnahme eines Schlüssels auf, wobei der Schlüssel an einer Bedieneinrichtung angeordnet ist und die Schnittstelle zur reversibel lösbaren Aufnahme der Bedieneinrichtung und zum Empfangen von Steuersignalen der Bedieneinrichtung ausgebildet ist. Der Schlüssel ist so mit der Bedieneinrichtung gekoppelt und repräsentiert die Identität der Bedieneinrichtung. Die Steuereinheit ist gemäß dieser Ausführungsform vorzugsweise dazu eingerichtet, die Freigabeeinrichtung in Abhängigkeit von der Identität der aufgenommenen Bedieneinrichtung zu steuern.

In einer vorteilhaften Ausgestaltung ist der Schlüssel als Daten-Schlüssel oder als Software-Schlüssel ausgebildet. Ein Daten-Schlüssel umfasst beispielweise eine Kodierung und ist beispielweise als 64-bit-Schlüssel ausgebildet. Er kann auch ein anderes geeignetes Datum, wie etwa ein Passwort, eine Kennung eines Bedieners, eine Kennung einer durchzuführenden Operation, eine Patientenerkennung oder dergleichen aufweisen. Die Schnittstelle ist in diesem Fall zum drahtgebundenen oder drahtlosen Empfang des Daten-Schlüssels ausgebildet und das Erfassungsmittel weist Softwaremittel zum Entschlüsseln des Schlüssels auf. Ein Software-Schlüssel umfasst bevorzugt ein Software-Modul mit Programmcode, welcher von der Steuereinheit in ein Programm aufgenommen, eingebettet oder von diesem verarbeitet wird. Ein solches Software-Modul kann etwa Programmanweisungen zur Steuerung des Haltearms, des Assistenzsystems und/oder eines an das Assistenzsystem angeschlossenen Anbaugeräts aufweisen.

Bevorzugt weist die Bedieneinheit des Haltearms eine Mehrzahl solcher Schnittstellen auf, vorzugsweise wenigstens eine, bevorzugt zwei Schnittstellen an jedem Armsegment. Bevorzugt ist der Haltearm grundsätzlich wie in DE 10 2014 016 824 der hiesigen Anmelderin offenbart ausgebildet, wobei die Bedieneinrichtung gemäß der vorliegenden Erfindung die Kontaktmittel des Haltearms gemäß DE 10 2014 016 824 aufweisen. Auf diese Weise ist der Haltearm gemäß der vorliegenden Erfindung dazu eingerichtet, bei Kontakt zwischen einer Bedienperson und einem der ersten und der zweiten Armsegmente, insbesondere einer an der entsprechenden Schnittstelle angeordneten Bedieneinrichtung, das jeweils zugeordnete Gelenk freizugeben. Bezüglich der weiteren Eigenschaften der Freigabe einzelner Gelenke durch Kontakt des zugeordneten Armsegments wird auf die Offenbarung von DE 10 2014 016 824 verwiesen.

Vorzugsweise sind an jedem Armsegment im Wesentlichen gegenüberliegend bezogen auf eine Längsachse des Armsegments zwei Schnittstellen ausgebildet sodass zwei Bedieneinrichtungen je Armsegment im Wesentlichen gegenüberliegend zueinander anordenbar sind. Dies hat den Vorteil, dass der Bediener das Armsegment nicht so genau kontaktieren muss, sondern es ausreicht, dass der Bediener das Armsegment im Wesentlichen umgreift und so mit den Bedieneinrichtungen in Kontakt kommt.

Gemäß einer weiteren bevorzugten Ausführungsform ist die Steuereinheit dazu eingerichtet, die Freigabeeinrichtung so in Anhängigkeit von der Identität des aufgenommenen Schlüssels zu steuern, dass bei Erfassen einer ersten Identität eines ersten Schlüssels eine erste Mehrzahl an Beschränkungen für den Haltearm vorgesehen ist, und dass bei Erfassen einer zweiten Identität eines zweiten Schlüssels eine zweite Mehrzahl an Beschränkungen für den Haltearm vorgesehen ist. Eine solche Mehrzahl an Beschränkungen kann beispielsweise eine Beschränkung in der Posen des Haltearms, in Winkelgeschwindigkeiten einzelner oder aller Gelenke, in Winkelbeschleunigungen einzelner oder aller Gelenke sowie eine Beschränkung des Rucks umfassen.

So kann beispielsweise vorgesehen sein, dass die erste Identität des ersten aufgenommenen Schlüssels angibt, dass es sich um einen Schlüssel für die Bauchchirurgie handelt. Dieser Schlüssel wird also von einem Operateur ausgewählt, der einen bauchchirurgischen Vorgang behandelt. Eine Beschränkung kann hier beispielsweise in Winkelgeschwindigkeiten aller Gelenke vorgesehen ist. Handelt es sich bei der Identität des Schlüssels allerdings um einen Schlüssel für die Kopfchirurgie, kann vorgesehen sein, dass die Winkelgeschwindigkeiten weiter auf einen sehr geringen Wert begrenzt sind, und dass zudem einzelne oder eine Mehrzahl von Posen beschränkt sind. Es kann zum Beispiel vorgesehen sein, dass auf Grund der Erfassung der Identität, eine Bewegung des Haltearms nur in einer Ebene möglich ist, und alle Posen, die außerhalb dieser Ebene liegen, nicht gestattet sind und die Steuereinheit die Freigabeeinrichtung entsprechend steuert. Das heißt, wenn ein Bediener in einem derartigen Fall versucht den Haltearm außerhalb der Ebene zu bewegen, steuert die Steuereinheit die Freigabeeinrichtung so, dass die Gelenke des Haltearms nicht freigegeben werden und eine Bewegung verhindert wird. Hierdurch ist die Sicherheit des Haltearms wesentlich erhöht und der Haltearm ist flexibel, durch die Wahl des entsprechenden Schlüssels an die Gegebenheiten bestimmter Operationsbereiche angepasst. Der Bediener wählt im Vorfeld der Operation einen Schlüssel, oder einen Satz von Schlüsseln, die dann vor Beginn der Operation in der entsprechenden Aufnahme bzw. den entsprechenden Aufnahmen des Haltearms anzuordnen sind. Das Erfassungsmittel der jeweiligen Schnittstelle erfasst die Identität des jeweils an dieser Schnittstelle aufgenommenen Schlüssels und übermittelt die Identität an die Steuereinheit. Die Steuereinheit verarbeitet diese Information entsprechend und empfängt oder ruft auf ein entsprechendes Programm, insbesondere Softwareprogramm, welches die mit den jeweiligen Identitäten verknüpften Beschränkungen und/oder Fähigkeiten des Haltearms umfasst. Ein solches Programm kann vorgespeichert sein und zu einer Mehrzahl an Identitäten vorgespeicherte Beschränkungen umfassen. Es kann auch vorgesehen sein, dass im Vorfeld zu einer Operation ein Operateur eine bestimmte Identität mit einer bestimmten Mehrzahl an Beschränkungen hinterlegt, um so den Haltearm für die geplante Operation vorzubereiten. Alternativ oder zusätzlich kann vorgesehen sein, dass das Erfassungsmittel solche Beschränkungen ebenfalls erfasst, wenn es die Identität des Schlüssels erfasst, die Beschränkungen also in dem Schlüssel gespeichert sind, und diese Information an die Steuereinheit übergeben wird. Während der Operation muss der Operateur dann nicht mehr darauf achten, dass der Haltearm irrtümlicherweise außerhalb der gewünschten Beschränkungen bewegt wird, da dies auf Grund der erfassten Identität mittels der Steuereinheit durch den Arm selbst gesperrt wird. Eine Fehlbewegung und dadurch Verletzung des Patienten ist verhindert

In einer weiteren bevorzugten Ausführungsform weist das Erfassungsmittel ein optisches Erfassungsmittel zum optischen Erfassen der Identität des Schlüssels auf. Ein solches optische Erfassungsmittel ist vorzugsweise als Kamera ausgebildet. Bevorzugt ist eine Kamera für den gesamten Haltearm vorgesehen, die sämtliche Schlüssel erfassen kann. Alternativ und bevorzugt ist an jedem Armsegment benachbart zu einer entsprechenden Schnittstelle eine Kamera vorgesehen, die Identität des jeweils an der Schnittstelle aufgenommenen Schlüssels erfassen kann. Eine derartige Kamera ist vorzugsweise mit einer Auswerteeinheit gekoppelt, die mittels Bildverarbeitung die Identität des aufgenommenen Schlüssels erfasst.

Die Auswerteeinheit kann im Haltearm oder außerhalb des Haltearms angeordnet sein. Vorzugsweise ist die Auswerteeinheit über ein Bussystem im Haltearm mit der oder den Kameras verbunden.

In einer bevorzugten Weiterbildung ist das optische Erfassungsmittel zum Erfassen einer farblichen Kodierung und/oder strukturellen Kodierung der Identität des Schlüssels ausgebildet. Es ist beispielsweise bevorzugt, dass der Schlüssel farblich kodiert ist. So kann beispielsweise für jede chirurgische Anwendung eine bestimmte Farbe vorgesehen sein. Beispielsweise sind Schlüssel für die Kopfchirurgie rot ausgebildet, während Schlüssel für die Bauchchirurgie grün ausgebildet sind. Dies hat gleichzeitig den Vorteil, dass diese Kodierung auch durch einen Operateur einfach erkennbar und verständlich ist, sodass er selbst ohne großen Aufwand überprüfen kann, ob der richtige Schlüssel ausgewählt wurde. Alternativ oder zusätzlich ist eine strukturelle Kodierung vorgesehen. Eine solche strukturelle Kodierung kann beispielsweise eine äußere Form, wie etwa oval oder rechteckig, umfassen, als auch eine Oberflächenstruktur oder speziell geformte Vorsprünge wie beispielsweise ein Vorsprung mit zwei, drei oder vier Zacken. Eine weitere Art der denkbaren Kodierung, ist eine mehrfarbige Kodierung, wie etwa ein Barcode, der aus schwarzen und weißen Strichen besteht. Ein solcher Barcode kann auch in farblichen, beispielsweise roten und weißen Strichen, grünen und weißen Strichen und dergleichen vorgesehen sein. Auch ein QR-Code, der so ausgebildet ist, ist bevorzugt. Vorteilhaft hieran ist, dass einerseits der Operateur auf Grund der Farbe den Schlüssel einfach zuordnen kann, zusätzlich können durch den Barcode oder den QR-Code erweiterte Informationen, wie beispielsweise eine erste Mehrzahl an Beschränkungen an das Erfassungsmittel übergeben werden.

In einer weiteren bevorzugten Ausgestaltung weist das Erfassungsmittel einen Empfänger zum Empfangen elektromagnetischer Wellen auf, wobei die elektromagnetischen Wellen die Identität des Schlüssels repräsentieren. Bei dieser Ausführungsform ist an dem oder als Schlüssel beispielsweise ein RFID-Transponder vorgesehen und ein entsprechendes RFID-Lesegerät ist im Bereich der Schnittstelle an dem jeweiligen Armsegment vorgesehen, sodass bei Aufnahme des Schlüssels der RFID-Transponder im Feld des Lesegeräts ist, und mittels des Lesegeräts ausgelesen wird. Das RFID-Lesegerät ist wiederum bevorzugt mit der Steuereinheit gekoppelt, beispielsweise über ein im Haltearm angeordnetes Bussystem. Auch hier liegt ein Vorteil darin, dass mittels des RFID-Transponders neben der reinen Identität des Schlüssels auch zusätzlich Information, wie beispielsweise Beschränkungen oder dergleichen übergeben werden können. Alternativ oder zusätzlich weist der Schlüssel einen NFC-Chip auf, über den der Schlüssel und ein im Haltearm angeordnetes entsprechendes Lesegerät kommunizieren können. Weist der Schlüssel zudem einen eigenen Mikrocontroller mit der entsprechenden Energieversorgung und Speichermitteln auf oder ist mit einem solchen verbunden, können über einen NFC-Chip auch Information und Daten von dem Haltearm an den Schlüssel und an mit diesem verbundenen Einrichtungen, wie beispielsweise der Bedieneinrichtung, übergeben werden.

In einer weiteren bevorzugten Ausführungsform weist das Erfassungsmittel eine elektronische Schnittstelle zum Empfangen eines Identifikationssignals des Schlüssels auf. Eine solche elektronische Schnittstelle kann beispielsweise als USB-Schnittstelle, Cinch-Schnittstelle oder dergleichen ausgebildet sein. Gemäß einer solchen Ausführungsform weist der Schlüssel vorzugsweise einen entsprechenden Stecker auf, der an einer solchen Schnittstelle einsteckbar ist. Auch hierüber ist die Identität des eingesteckten Schlüssels auf einfach Art und Weise an den Haltearm übermittelbar. Gleichzeitig bietet eine solche Schnittstelle auch eine gewisse mechanische Kopplung und beim Anordnen des Schlüssels an der Schnittstelle des Haltearms und eine mechanische Rückkopplung über die Verbindung.

Gemäß einer weiteren bevorzugten Ausführungsform weist das Erfassungsmittel ein elektromechanisches Schloss zur Aufnahme einer Mehrzahl an Schlüsseln auf, wobei die Steuereinheit dazu eingerichtet ist, die Freigabeeinrichtung in Anhängigkeit von dem aufgenommenen Schlüssel zu steuern. Beispielsweise bevorzugt ist eine gestufte Vertiefung in der Schnittstelle an dem Haltearm vorgesehen, in die ein entsprechender Vorsprung des Schlüssels einsteckbar ist. In der gestuften Vertiefung sind mehrere elektrische Schalter angeordnet, die je nach Ausbildung des Vorsprungs des Schlüssels einen elektrischen Kontakt schließen. Abhängig von der Kombination der geschlossenen elektrischen Kontakte ist die Identität des Schlüssels erkennbar. Dies ist eine besonders einfache Möglichkeit die Identität zu kodieren und zu erfassen. Ein solches elektromechanisches Schloss kann bevorzugt als Stiftschloss ausgebildet sein, wobei die einzelnen Stifte in Abhängigkeit von der Ausprägung des Schlüssels ausgelenkt werden und diese Auslenkung von einem Erfassungsmittel erfasst wird und dadurch die Identität des Schlüssels erfasst wird. Dies ist eine weitere Möglichkeit der einfachen mechanischen Kodierung, die zudem eine Vielzahl an Varianten zulässt, sodass für eine Vielzahl verschiedener Operationen und Operationsbedingungen eine Vielzahl an Schlüsseln codiert werden können.

Gemäß einer weiteren bevorzugten Ausführungsform weist der Haltearm eine sterile Tüte auf, die den Haltearm umgibt, wobei die Tüte im Bereich der Schnittstelle zur reversibel lösbaren Aufnahme einer Bedieneinrichtung und/oder zur Aufnahme eines Schlüssels eine Ausnehmung aufweist und/oder durchstechbar und/oder von elektromagnetischen Wellen durchdringbar ausgebildet ist. Auf all diese Weisen ist es möglich, den Haltearm zunächst mit einer sterilen Tüte zu verkleiden und anschließend einen entsprechenden Schlüssel und/oder eine entsprechende Bedieneinrichtung an dem Haltearm anzuordnen. Hierdurch wird die Handhabung wesentlich vereinfacht. Die Bedieneinrichtung kann außerhalb der Tüte sein und dennoch in Wirkverbindung mit dem Haltearm stehen, sodass der Haltearm mittels der Bedieneinrichtung bzw. den Bedieneinrichtungen bedienbar ist. Hierdurch ist die Sicherheit des Haltearms verbessert.

Weiterhin ist bevorzugt, dass die Bedieneinrichtung und/oder die Schnittstelle an dem Assistenzsystem angeordnet ist. Ein solches Assistenzsystem kann beispielsweise als sogenannter Manipulator ausgebildet sein, der dazu vorgesehen ist ein Kinematik-Modul aufzunehmen. Ein Kinematik-Modul kann beispielsweise ein medizinisches Instrument, wie etwa ein Endoskop oder eine Biopsienadel oder dergleichen halten. Das Kinematik-Modul weist in dieser Ausführungsform vorzugsweise den Schlüssel auf und dieser wird durch Anordnen, insbesondere Einstecken, des Kinematik-Moduls an den Manipulator in der an dem Manipulator ausgebildeten Schnittstelle aufgenommen. Durch Erfassen der Identität des Schlüssels kann dann beispielsweise erfasst werden, ob das Kinematik-Modul neu oder gebraucht ist, wieviele Betriebsstunden es verwendet wurde, oder ob es ausreichend sterilisiert wurde. Hierdurch wird die Sicherheit wesentlich verbessert.

Gemäß einem zweiten Aspekt der Erfindung wird die eingangs genannte Aufgabe gelöst durch eine Bedieneinrichtung für einen Haltearm nach einer der vorstehend beschriebenen bevorzugten Ausführungsformen eines Haltearms gemäß dem ersten Aspekt der Erfindung, wobei die Bedieneinrichtung einen Schlüssel für die Schnittstelle zur Aufnahme eines Schlüssels aufweist. Die Bedieneinrichtung weist den Schlüssel auf und ist vorzugsweise als Schlüssel ausgebildet. Neben dem Schlüssel kann die Bedieneinrichtung weitere Elemente und Einrichtungen zur Bedienung des Haltearms aufweisen. Der Schlüssel wird, sofern dieser in beziehungsweise an der Schnittstelle zur Aufnahme des Schlüssels angeordnet ist, durch das Erfassungsmittel erfasst, sodass die Identität des Schlüssels erfasst wird und die Steuereinheit die Freigabeeinrichtung in Abhängigkeit dieser Identität steuert.

Es soll verstanden werden, dass der Haltearm gemäß dem ersten Aspekt der Erfindung und die Bedieneinrichtung gemäß dem zweiten Aspekt der Erfindung gemeinsame bevorzugte Ausführungsformen und Weiterbildungen aufweisen, wie sie insbesondere in den Unteransprüchen niedergelegt sind. Insbesondere werden die Vorteile der Erfindung durch eine Kombination des Haltearms gemäß dem ersten Aspekt der Erfindung und der Bedieneinrichtung gemäß dem zweiten Aspekt der Erfindung verkörpert, wenn diese zusammenwirken. Insofern wird vollumfänglich auf die obige Beschreibung und die dargelegten Vorteile verwiesen, wobei beide Elemente, der Haltearm und die Bedieneinrichtung, sowohl unabhängig voneinander beansprucht werden als auch gemeinsam als System.

Gemäß einer ersten bevorzugten Ausführungsform der Bedieneinrichtung weist diese einen Grundkörper, eine Eingabeeinrichtung zum Eingeben eines Steuersignals durch einen Bediener und eine Schnittstelle zum Übertragen des Steuersignals an den Haltearm auf, wobei der Schlüssel eine Identität der Bedieneinrichtung angibt und mittels des Erfassungsmittels des Haltearms erfassbar ist. Der Schlüssel kodiert gemäß diesem Ausführungsbeispiel die Identität der Bedieneinrichtung. Die Bedieneinrichtung weist vorzugsweise eine Kontaktfläche, wie oben beschrieben, und wie in DE 10 2014 016 824 offenbart auf. Die Bedieneinrichtung ist demnach samt der daran angeordneten Kontaktfläche an dem Haltearm anordenbar, insbesondere in diesen einsteckbar, um so eine Bedienung des Haltearms zu ermöglichen. Indem die Bedieneinrichtung neben dem Schlüssel auch die Eingabeeinrichtung, die vorzugsweise als berührungsempfindliche Kontaktfläche ausgebildet ist, zum Eingeben eines Steuersignals durch den Bediener aufweist, ist auch die Eingabeeinrichtung samt der Bedieneinrichtung von dem Haltearm reversibel abnehmbar, sodass der Haltearm ohne die daran angeordnete Bedieneinrichtung bzw. Bedieneinrichtungen nicht betreibbar ist. Dies erhöht wiederum die Sicherheit. Ferner ist es so möglich, auch die Eingabeeinrichtung auf einen speziellen Operationsbereich abzustimmen. Beispielsweise kann es erforderlich sein, für den Bereich der Kopfchirurgie eine abweichend ausgebildete Eingabeeinrichtung vorzusehen als für den Bereich der Bauchchirurgie. Zudem kann so die Bedieneinrichtung samt der Eingabeeinrichtung separat sterilisiert werden, was ebenfalls der Sicherheit dient.

In einer bevorzugten Weiterbildung ist vorgesehen, dass der Schlüssel eine optische Kodierung aufweist. Der Schlüssel kann gemäß diesem Ausführungsbeispiel als farbliche Fläche ausgebildet sein, die auf einer Oberseite der Bedieneinrichtung vorgesehen ist. Weiterhin kann vorgesehen sein, dass der Schlüssel als ein Barcode oder QR-Code ausgebildet ist. Bevorzugt ist auch eine Kombination von einer farblichen Flächenkodierung und einem Bar- oder QR-Code. Weiterhin ist bevorzugt, dass der Schlüssel eine optisch erfassbare strukturelle Kodierung aufweist, wie beispielsweise eine Oberflächenstruktur, eine Form, eine Kontur oder ein Vorsprung mit bestimmter Form.

Gemäß einer weiteren bevorzugten Ausführungsform weist der Schlüssel einen Sender zum Senden elektromagnetischer Wellen auf. Beispielsweise weist der Schlüssel einen RFID-Transponder auf, der, wenn er sich in einem elektromagnetischen Feld eines RFID-Lesegeräts befindet, elektromagnetische Wellen sendet, die die Identität der Bedieneinrichtung repräsentieren. Ist die Bedieneinrichtung an der entsprechenden Schnittstelle am Haltearm angeordnet, ist der RFID-Transponder durch das Lesegerät auslesbar und die Identität der Bedieneinrichtung feststellbar.

Bevorzugt ist auch, dass der Schlüssel eine elektronische Schnittstelle zum Senden eines Identifikationssignals aufweist. Der Schlüssel kann gemäß einer derartigen Ausführungsform beispielsweise als Chip ausgebildet sein, der mit einem USB-Anschluss, einem Chinch-Anschluss oder dergleichen ausgestattet ist. Auch andere Schnittstellen sind hier denkbar.

Gemäß einer weiteren bevorzugten Ausführungsform ist der Schlüssel als mechanisch einsteckbarer Schlüssel ausgebildet. Der Schlüssel weist vorzugsweise eine strukturierte Oberfläche auf, mittels der die Identität der Bedieneinrichtung kodiert ist. Der Schlüssel weist vorzugsweise einen Schlüsselbart auf, der mittels eines entsprechenden Stiftschlosses am Haltearm abtastbar und in ein elektronisches Signal umwandelbar ist.

In einer weiteren bevorzugten Ausgestaltung weist die Bedieneinrichtung eine Schnittstelle für einen optischen Reflektor für eine chirurgische Navigation auf. Derartige Reflektoren werden von herkömmlichen OP-Navigationssystemen verwendet, um einzelne Instrumente, Geräte oder spezifische Punkte zu identifizieren und in die Navigation einzubinden. Vorzugsweise ist an jedem Armsegment eine Bedieneinrichtung angeordnet, die eine Schnittstelle für einen solchen Reflektor aufweist. So ist jedes Armsegment des Haltearms, mit dem eine Bedieneinrichtung gemäß dem zweiten Aspekt der Erfindung angeordnet ist, in das Navigationssystem einbindbar. Dies ist besonders bevorzugt, da die Bedieneinrichtung außerhalb einer sterilen Tüte, die den Haltearm umgibt, angeordnet ist. Dadurch ist auch die Schnittstelle für den Reflektor außerhalb der Tüte, und der Reflektor kann ohne Weiteres und ohne zusätzliche Verletzung der Tüte an dem Haltearm angeordnet werden.

Bevorzugt ist ferner, dass die Bedieneinrichtung Anzeigemittel aufweist, vorzugsweise Beleuchtungsmittel, zum Anzeigen eines Zustands der Bedieneinrichtung und/oder des Haltearms und/oder eines an dem Haltearm angeschlossenen mechatronischen Assistenzsystems und/oder chirurgischen Instruments. Zum einen ist bevorzugt, dass die Anzeigemittel, die bevorzugterweise als Beleuchtungsmittel ausgebildet sind, einen Zustand der Bedieneinrichtung anzeigen. Ein solcher Zustand kann zum einen sein, dass die Bedieneinrichtung korrekt mit der Schnittstelle des Haltearms verbunden ist. Ein anderer Zustand kann sein, dass die Bedieneinrichtung korrekt von dem Haltearm beziehungsweise von dem Erfassungsmittel des Haltearms identifiziert und einer bestimmten vorbekannten Mehrzahl an Beschränkungen zugeordnet wurde. Die Beleuchtungsmittel sind bevorzugt als farbige LED ausgebildet, die über Aufleuchten und Blinken den Zustand anzeigen. Ferner werden derartige Anzeigemittel bevorzugt genutzt, einen Zustand des Haltearms anzuzeigen. Beispielsweise zeigen die Anzeigemittel an, ob der Haltearm korrekt funktioniert oder ob ein Fehler festgestellt wurde. Sie können auch eine Überlastung des Haltearms oder die Kollision mit einer vorbestimmten Operationsfeldbeschränkung anzeigen. Darüber hinaus zeigen die Anzeigemittel vorzugsweise den Zustand eines an dem Haltearm angeschlossenen mechatronischen Assistenzsystems und/oder chirurgischen Instruments an. Auch hier können die Anzeigemittel anzeigen, ob das Assistenzsystem korrekt mit der entsprechenden Schnittstelle am distalen Ende des Haltearms gekoppelt wurde.

Der Haltearm weist dazu vorzugsweise ein Bussystem auf, an welches das Assistenzsystem angekoppelt ist und über das Daten ausgetauscht werden. An dieses Bussystem ist sowohl die Steuereinheit als auch das Erfassungsmittel angeschlossen, sowie die Schnittstelle für die Bedieneinrichtung. Weist die Bedieneinrichtung entsprechende Kommunikationsmittel auf wie beispielsweise einen NFC-Chip, können Daten und Informationen zwischen der Bedieneinrichtung und dem Haltearm ausgetauscht werden, sodass ein Zustand des Assistenzsystems über das Bussystem, die NFC-Verbindung an die Bedieneinrichtung übergeben werden kann, die dann mittels der Anzeigemittel den Zustand des Assistenzsystems anzeigt.

In einer weiteren bevorzugten Ausführungsform weist die Bedieneinrichtung einen Bildschirm zum Anzeigen von Daten und/oder eines Patientenmodells auf. Solche Daten können beispielsweise eine Eindringtiefe eines Endoskops, einen Saugdruck eines Saugers, Gewichtskräfte am distalen Ende des Haltearms oder dergleichen umfassen. Sämtliche für die Ausführung der Operation erforderliche Daten können auf dem Bildschirm angezeigt werden. Weiterhin ist es möglich, ein Patientenmodell auf dem Bildschirm anzuzeigen, auf dem insbesondere die Lage des Haltearms relativ zum Patienten oder eines am distalen Ende angeordneten Assistenzsystems oder Instruments zum Patienten dargestellt ist. Hierdurch ist der Bildschirm nah am Operationsfeld, und der Operateur kann seinen Blick in Richtung des Operationsfelds richten und so sowohl das Operationsfeld als auch den Bildschirm mit den wichtigsten Daten sehen. Hierdurch kann vermieden werden, dass der Operateur häufig seinen Kopf wenden muss, um vom Operationsfeld auf einen entfernt angeordneten Bildschirm zu schauen, was auch stets eine Anpassung der Tiefenschärfe mit den Augen umfasst, was rasch zu Ermüdung führen kann.

Gemäß einer weiteren bevorzugten Ausführungsform weist die Bedieneinrichtung ein Vibrationsmodul auf, über das die Bedieneinrichtung in Vibration versetzbar ist. Die Bedieneinrichtung ist somit in der Lage, dem Bediener ein taktiles Feedback zu geben. Beispielsweise kann vorgesehen sein, dass das Vibrationsmodul für einen kurzen Moment, beispielsweise eine halbe oder eine Viertelsekunde, in Vibration versetzt wird, wenn der Bediener eine Kontaktfläche einer Bedieneinrichtung so berührt, dass ein Signal von der Bedieneinrichtung an die Steuereinheit des Haltearms zum Freigeben eines Gelenks gesendet wird. Alternativ kann vorgesehen sein, dass die Bedieneinrichtung dann in Vibration versetzt wird, wenn der Bediener versucht, eine nicht erlaubte Operation mit dem Haltearm durchzuführen, beispielsweise den Haltearm versucht, in eine Pose zu verbringen, die aufgrund der ermittelten Identität der Bedieneinrichtung nicht gestattet ist. Dies erhöht die Sicherheit und gibt dem Operateur zusätzlich Rückmeldung über die von ihm vorgenommenen Handlungen.

In einer besonders bevorzugten Ausführungsform weist die Eingabeeinrichtung der Bedieneinrichtung eine berührungsempfindliche Fläche zur Steuerung des Haltearms auf. Vorzugsweise ist die berührungsempfindliche Fläche entsprechend der Kontaktfläche gemäß DE 10 2014 016 824 und wie zusätzlich hierin weiter unten beschrieben ausgebildet. Die Bedieneinrichtung umfasst gemäß diesem Ausführungsbeispiel vorzugsweise auch eine Sensorik, die die Berührung erkennt. Die berührungsempfindliche Fläche ist beispielsweise als Touchpad oder Touch-Display ausgebildet.

Alternativ ist die berührungsempfindliche Fläche mit einem Fluidkörper ausgebildet. Beispielsweise weist diese eine Kavität auf, die auf einer nach außen gerichteten Seite mit einer elastischen Schicht geschlossen ist. In der Kavität ist ein Gel oder ein anderes Fluid angeordnet sowie ein oder mehrere Drucksensoren. Berührt nun ein Operateur die elastische Oberfläche, wird der Druck innerhalb der Kavität erhöht, was durch den einen oder die mehreren Drucksensoren erfasst wird. Das hieraus resultierende Signal wird als Steuersignal verwendet, welches an die Steuereinheit im Haltearm mittels des Bussystems im Haltearm weitergeleitet wird. Aufgrund dieses Steuersignals wird dann ein Gelenk freigegeben oder gesperrt. Ein solches System mit einer Fluidkammer hat den Vorteil, dass die einzelnen Komponenten preiswert sind, und dass die Bedieneinrichtung, die so ausgebildet ist, auf einfache Art und Weise sterilisierbar ist. Ferner ist ein solches System wenig fehleranfällig und leicht skalierbar. Unabhängig davon, an welcher Stelle der elastischen Schicht der Operateur die Eingabeeinrichtung berührt, wird ein entsprechendes Signal durch den einen oder die mehreren Drucksensoren erzeugt.

Gemäß einer weiteren bevorzugten Ausführungsform weist die Bedieneinrichtung eine elektronische Spule für eine chirurgische Navigation auf. Die Spule wird bevorzugt über eine Schnittstelle zum Arm angebunden sowie die Schnittstelle des Haltearms am proximalen Ende an ein OP-System und darüber an die übrigen OP-Geräte des Operationssaals, wie insbesondere einem elektromagnetischen Navigationssystem für den Operationssaal. Damit wird eine Navigation einzelner Segmente des Haltearms oder des gesamten Haltearms in einer elektromagnetischen Navigationsumgebung möglich.

Gemäß einer weiteren bevorzugten Ausführungsform weist die Eingabeeinrichtung einen kapazitiven Sensor auf, der dazu ausgebildet ist, eine Abstandsänderung der Bedieneinrichtung zum Haltearm zu erfassen und in ein elektrisches Signal zu wandeln. Es ist dabei nicht erforderlich, dass die gesamte Bedieneinrichtung im Abstand zum Haltearm geändert wird, sondern auch ein Teil von dieser, wie beispielsweise eine Oberfläche. Beispielsweise weist die Eingabeeinrichtung eine Oberfläche auf, die elastisch ist und die mit dem kapazitiven Sensor zusammenwirkt. Bei Aufbringen von Druck gegen diese elastische Oberfläche wird diese leicht in Richtung des Haltearms verformt, wobei der kapazitive Sensor ein Signal erzeugt, welches über die Schnittstelle und das interne Bussystem des Haltearms an die Steuereinheit weitergegeben wird, die aufgrund dieses Signals ein Gelenk freigibt oder sperrt. Eine derartige Ausgestaltung hat den Vorteil, dass die Eingabeeinrichtung eine geschlossene Oberfläche aufweist, wodurch wiederum die Sterilisation vereinfacht ist.

Vorzugsweise oder alternativ weist die Bedieneinrichtung auf ihrer Unterseite zum Haltearm hin oder an einem mechanischen Zapfen, mittels dem die Bedieneinrichtung mit dem Haltearm verbunden wird, einen oder mehrere zusätzliche Sensoren auf. Im Haltearm ist ein entsprechender Elektronikbaustein vorgesehen. Zusammen mit dem Sensor und der Außenhülle des Haltearms bildet die Bedieneinrichtung eine kapazitive Abstandsmessung. Auch durch eine solche Ausgestaltung können kleinste Bewegungen der auf den Haltearm aufgesteckten Bedieneinrichtung erkannt und in ein Steuersignal umgewandelt werden.

Ist eine Bedieneinrichtung, die den Schlüssel aufweist an einer entsprechenden Schnittstelle des Haltearms angeordnet, oder ist eine Mehrzahl an Bedieneinrichtungen, insbesondere zwei je Armsegment, angeordnet ergibt sich ein System, welches die oben beschriebenen Vorteile kombiniert und die in DE 10 2014 016 824 beschriebenen Effekte erreicht. Insbesondere kann vorgesehen sein, dass die Bedieneinheit dazu ausgebildet, in Abhängigkeit der Intensität des Kontakts das zugeordnete Gelenk freizugeben. Intensität bezieht sich hier auf einen Druck und/oder eine Kraft, der bzw. die durch den Bediener aufgebracht wird. So ist es möglich, dass der Bediener durch die Kraft, die er beim Zugreifen aufbringt, einen Grad der Freigabe steuert. So ist es denkbar und bevorzugt, dass das zugeordnete Gelenk bei einer geringen Intensität des Kontakts nur teilweise freigegeben wird, sodass das Armsegment nur langsam und gegen einen Widerstand bewegbar ist. Bei einer hohen Intensität und demzufolge einem festen Zugreifen wird das Gelenk dann vollständig geöffnet, sodass das Armsegment im Wesentlichen widerstandsfrei beweglich ist. Ein teilweises Freigeben kann auch durch ein intermittierendes Freigeben in verschiedenen Frequenzen realisiert werden. Bevorzugt ist vorgesehen, dass das erste Gelenk an einem proximalen Ende des ersten Armsegments angeordnet ist und dass das zweite Gelenk an einem proximalen Ende des zweiten Armsegments angeordnet ist. Jedes Armsegment hat ein proximales und ein distales Ende, wobei das proximale Ende des Armsegments dasjenige Ende ist, welches in Armrichtung proximal zum proximalen Ende des Haltearms liegt, und das distale Ende des Armsegments dasjenige Endet ist, das entlang des Haltearms zum distalen Ende hin ausgerichtet ist.

Vorzugsweise weisen die Gelenke des Haltearms Bremsen auf, mittels derer die Gelenke freigebbar und arretierbar sind. Der Haltearm ist vorzugsweise als passiver Haltearm ausgebildet. Die Bremsen dienen dazu, eine Bewegung der Armsegmente relativ zueinander, also eine Bewegung der Gelenke zu bremsen beziehungsweise zu verhindern. Sind die Bremsen gelöst, sind die Gelenke freigegeben. Vorzugsweise sind die Bremsen in einem Ruhezustand derart vorgespannt, dass die Gelenke arretiert sind. Besonders bevorzugt sind die Bremsen als elektromagnetische Bremsen ausgebildet und weisen jeweils einen Permanentmagneten auf, der die Bremsen im unbestromten Zustand in den arretierten Zustand vorspannt.

Gemäß einer weiteren bevorzugten Ausgestaltung weist der Haltearm sechs Freiheitsgrade auf. Besonders bevorzugt weist der Haltearm sieben Freiheitsgrade auf. Während sechs Freiheitsgrade ausreichen, um jeden Punkt im Raum zu erreichen, ist es bei sieben Freiheitsgraden möglich, jeden Punkt mit verschiedenen Posen zu erreichen, sodass der Haltearm stets so ausrichtbar ist, dass beispielsweise das Operationsfeld leicht zugänglich ist. Daher ist es besonders bevorzugt, dass der Haltearm sieben Freiheitsgrade aufweist. Gemäß einer bevorzugten Ausführungsform weist der Haltearm sieben Armsegmente und sieben Gelenke auf, wobei jedem Armsegment ein Gelenk zugeordnet ist. Jedes Gelenk weist gemäß diesem Ausführungsbeispiel vorzugsweise einen Freiheitsgrad auf, sodass der Haltearm insgesamt sieben Freiheitsgrade aufweist. Es ist auch möglich, dass jedes Gelenk zwei oder mehr Freiheitsgrade hat, wobei Gelenke mit einem Freiheitsgrad aufgrund ihrer Stabilität bevorzugt sind. Vorzugsweise sind sämtliche Gelenke als rotatorische Gelenke ausgebildet. Vorzugsweise sind einige der Gelenke als rotatorische Gelenke und einige als translatorische Gelenke ausgebildet. Vorzugsweise sind die Gelenke, wenn diese sämtlich rotatorisch ausgebildet sind, derart in dem Haltearm angeordnet, dass Achsen von entlang des Haltearms aufeinanderfolgenden Gelenken, vom proximalen zum distalen Ende des Haltearms hin, jeweils senkrecht aufeinander stehen. Vorzugsweise weist der Haltearm eine Gewichtskompensationseinrichtung zum wenigstens teilweisen Abstützen des Gewichts eines oder mehrerer Armsegmente des Haltearms auf, wenn ein oder mehrere Gelenke freigegeben sind.

Ferner kann vorgesehen sein, dass an den Armsegmenten Ausrichtungsindikatoren angeordnet sind, die eine Grundpose des Haltearms angeben.

Weiterhin ist bevorzugt, dass innerhalb der Armsegmente wenigstens ein Kabelkanal zum Durchführen von Kabeln von dem proximalen zum distalen Ende des Haltearms vorgesehen ist. In einer weiteren bevorzugten Ausführungsform weist das erste Armsegment, bezogen auf das proximale Ende des Haltearms, erste mechanische Kopplungsmittel zum lösbaren Koppeln des Haltearms mit zweiten korrespondierenden Kopplungsmitteln eines Operationstisches auf. Weiterhin ist bevorzugt, dass das letzte Armsegment am distalen Ende des Haltearms eine mechatronische Schnittstelle zum Koppeln des chirurgischen mechatronischen Assistenzsystems und/oder chirurgischen Instruments mit dem Haltearm aufweist. Eine derartige mechatronische Schnittstelle weist vorzugsweise mechanische Kopplungsmittel zum mechanischen Halten des Assistenzsystems und/oder chirurgischen Instruments auf, und andererseits elektronische Schnittstellen zum Übertragen von elektrischer Energie und/oder Daten beziehungsweise Signalen an das mechatronische Assistenzsystem.

Nachfolgend wird die Erfindung anhand von vier Ausführungsbeispielen unter Bezugnahme auf die beiliegenden Figuren genauer beschrieben werden. Dabei zeigen:
- Figur 1: eine Seitenansicht eines Haltearms, bei dem die Kontaktmittel zu sehen sind;
- Figur 2: eine teilweise aufgebrochene Ansicht des Haltearms aus Figur 1;
- Figur 3: eine schematische Darstellung des vierten Armsegments;
- Figur 4: drei Haltearmsegmente mit Schnittstelle, Schlüssel und Bedieneinrichtung;
- Figur 5: ein Haltearmsegment mit einer Schnittstelle und einem Schlüssel gemäß einem ersten Ausführungsbeispiel;
- Figur 6: ein Haltearmsegment mit einer Schnittstelle und einem Schlüssel gemäß einem zweiten Ausführungsbeispiel;
- Figur 7: ein Haltearmsegment mit einer Schnittstelle und einem Schlüssel gemäß einem dritten Ausführungsbeispiel;
- Figur 8: ein Haltearmsegment mit einer Schnittstelle und einem Schlüssel, bei dem eine kapazitive Abstandsmessung vorgesehen ist; und
- Figur 9: einen Haltearm gemäß einem zweiten Ausführungsbeispiel.

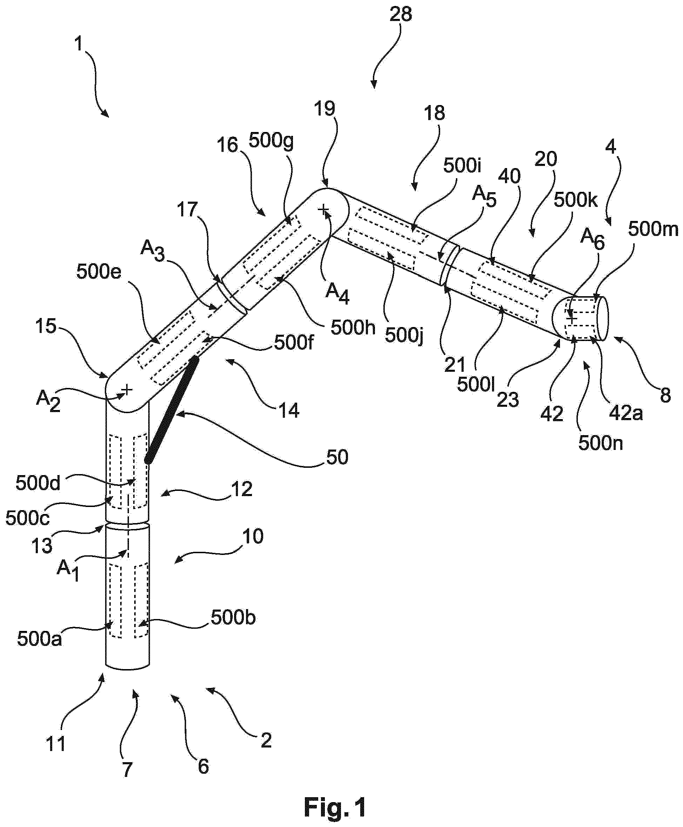

Figur 1 zeigt einen Haltearm 1 für medizinische Zwecke, insbesondere zum Halten eines chirurgischen mechatronischen Assistenzsystems und/oder chirurgischen Instruments. Der Haltearm 1 weist ein proximales Ende 2 und ein distales Ende 4 auf. An dem proximalen Ende 2 sind eine erste Schnittstelle 6 und eine mechanische Schnittstelle 7 ausgebildet. Die Schnittstelle 7 dient dazu, den Haltearm 1 an einer Basis, wie insbesondere an einem Operationstisch, zu befestigen. Die Schnittstelle 6 dient zur Übergabe von Energie sowie zur Kopplung des Haltearms 1 mit einer externen Steuereinheit. An dem distalen Ende 4 ist eine zweite Schnittstelle 8 vorgesehen, über die ein mechatronisches Assistenzsystem und/oder ein chirurgisches Instrument, wie insbesondere ein Manipulator, an den Haltearm 1 koppelbar ist. Vorzugsweise wird hier ein Manipulator zum Halten und Manipulieren eines Endoskops angeordnet.

Der Haltearm 1 gemäß Figur 1 weist sieben Armsegmente 10, 12, 14, 16, 18, 20, 22 auf, die jeweils im Wesentlichen stabförmig sind und bis auf das letzte Armsegment 22 alle im Wesentlichen eine gleiche Länge aufweisen. Die sieben Armsegmente 10, 12, 14, 16, 18, 20, 22 sind jeweils mit Gelenken 11, 13, 15, 17, 19, 21, 23 miteinander gekoppelt, wobei das nullte Gelenk 11 den Haltearm 1 mit der Basis (in Fig. 1 nicht gezeigt) koppelt. Die Gelenke 13, 15, 17, 19, 21, 23 sind gemäß diesem Ausführungsbeispiel sämtlich als rotatorische Gelenke mit jeweils einem Freiheitsgrad ausgebildet. Gemäß diesem Ausführungsbeispiel ist dem nullten Segment 10 das nullte Gelenk 11 zugeordnet, dem ersten Armsegment 12 das erste Gelenk 13 zugeordnet, dem zweiten Armsegment 14 das zweite Gelenk 15 zugeordnet, dem dritten Armsegment 16 das dritte Gelenk 17 zugeordnet, dem vierten Armgelenk 18 das vierte Gelenk 19 zugeordnet, dem fünften Armsegment 20 das fünfte Gelenk 21 zugeordnet, und dem sechsten Armsegment 22 ist das sechste Gelenk 23 zugeordnet. Das Gelenk 11 ist als translatorisches Gelenk ausgebildet, sodass das Armsegment 10 teleskopartig verlängerbar ist, um so den Haltearm 1 in der Höhe zu verstellen. Die Gelenke 13, 15, 17, 19, 21, 23 weisen jeweils Schwenkachsen A₁, A₂, A₃, A₄, A₅, A₆ auf, wobei jeweils benachbarte Gelenke Schwenkachsen haben, die senkrecht zueinander sind. Hierdurch wird eine einfache Positionierung des distalen Endes 4 im Raum erreicht.

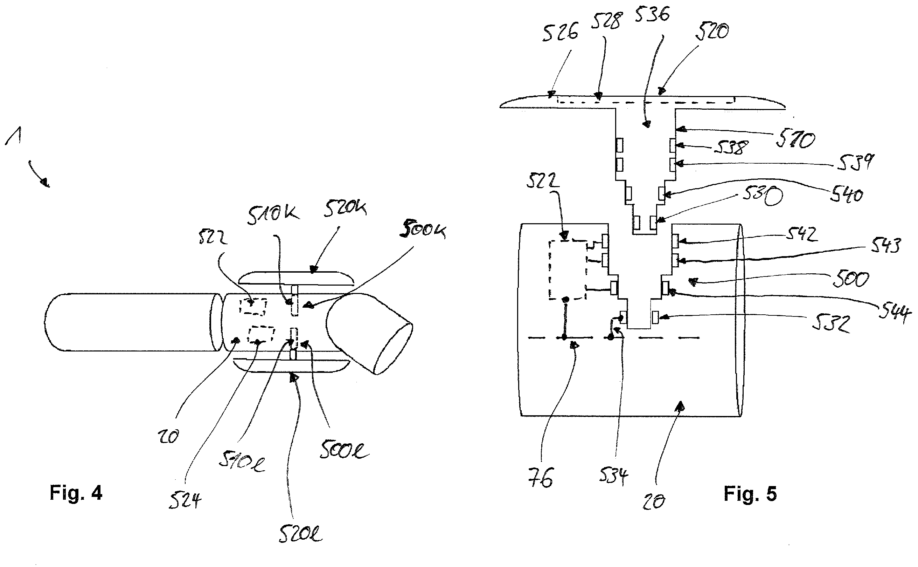

Der Haltearm 1 gemäß Figur 1 weist ferner eine Bedieneinheit 28 auf. Mittels der Bedieneinheit 28 ist der Haltearm 1 in eine gewünschte Pose verbringbar, wobei die Bedieneinheit 28 dazu eingerichtet ist, bei Kontakt zwischen einer Bedienperson und einem der sieben Armsegmente das zugeordnete Gelenk freizugeben. Dazu weist die Bedieneinheit 28 gemäß diesem Ausführungsbeispiel sieben paare an Schnittstellen 500a bis 500n (insgesamt mit 500 bezeichnet) zur Aufnahme eines Schlüssels (vgl. Fig. 5) auf. An jeder dieser Schnittstellen 500 ist jeweils ein Schlüssel aufnehmbar, wobei die Schnittstellen gleichzeitig als Schnittstellen für entsprechend vierzehn Bedieneinrichtungen (vgl. Fig. 5-7) dienen.

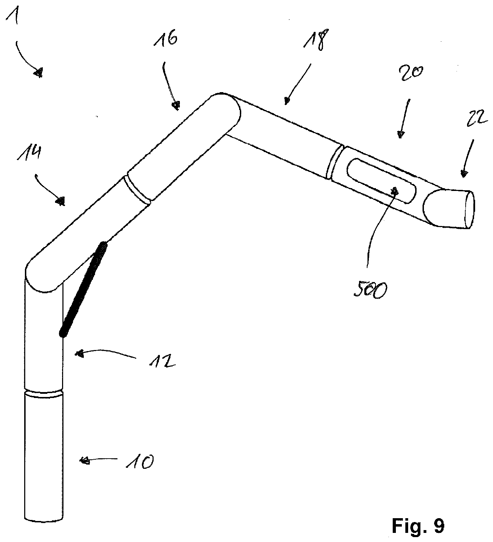

In einer anderen Ausführungsform weist der Haltearm 1 nur eine Schnittstelle (500) für nur einen Schlüssel 510 auf (vgl. Figur 9).

Weiterhin ist in der Figur 1 zu erkennen, dass der Haltearm 1 über eine Gewichtskompensationseinrichtung 50 verfügt. Die Gewichtskompensationseinrichtung 50 weist gemäß diesem Ausführungsbeispiel ein Gasdruckfederelement auf, welches mit dem Armsegment 14 und dem Armsegment 12 gekoppelt ist. Alternativ kann die Gewichtskompensationseinrichtung auch einen Seilzug und/oder ein ausbalanciertes Gegengewicht aufweisen. Bei dem Haltearm 1 gemäß Figur 1 lastet auf dem Gelenk 15 um seine Rotationsachse A2 das größte Moment. Folglich ist es bevorzugt, genau dieses Gelenk 15 mittels der Gewichtskompensationseinrichtung 50 abzustützen. Bei Freigabe des Gelenks 15, durch Kontaktieren des Armsegments 14, wird also ein Gewicht, welches auf dem Armsegment 14 lastet, aufgrund der weiteren Armsegmente 16, 18, 20, 22 und eines an der Schnittstelle 8 angeordneten Manipulators durch die Gewichtskompensationseinrichtung 50 abgestützt, sodass das distale Ende 4 bei Ergreifen des Segments 14 nicht sofort "absackt".

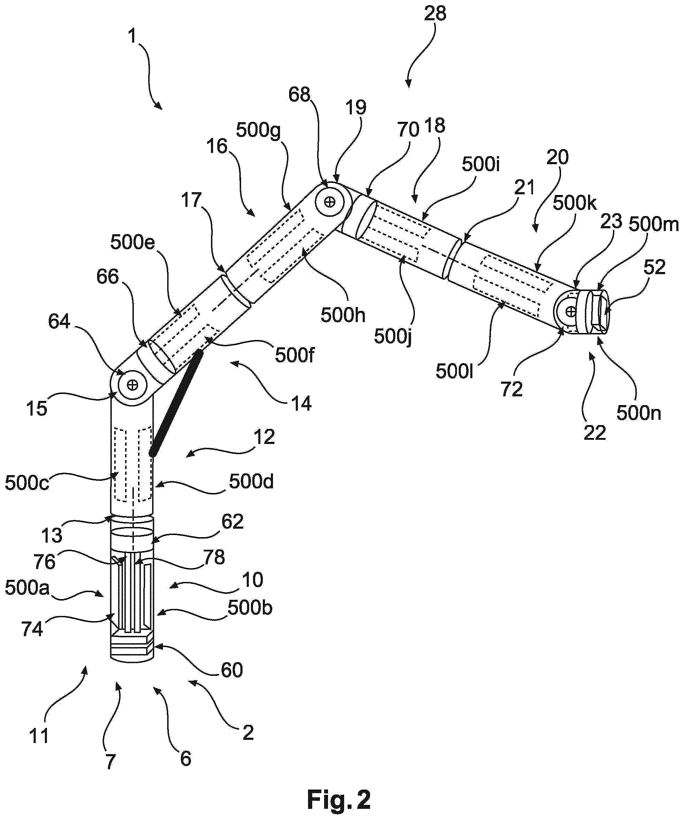

In Figur 2 sind bei dem Haltearm 1 zusätzlich zu den bereits in Figur 1 gezeigten Elementen die Bremsen 60, 62, 64, 66, 68, 70, 72 dargestellt, mittels derer die Gelenke 11, 13, 15, 17, 19, 21, 23 freigebbar und arretierbar sind. Gleiche und ähnliche Elemente sind mit gleichen Bezugszeichen wie in Figur 1 versehen, und insofern wird vollumfänglich auf die obige Beschreibung Bezug genommen.

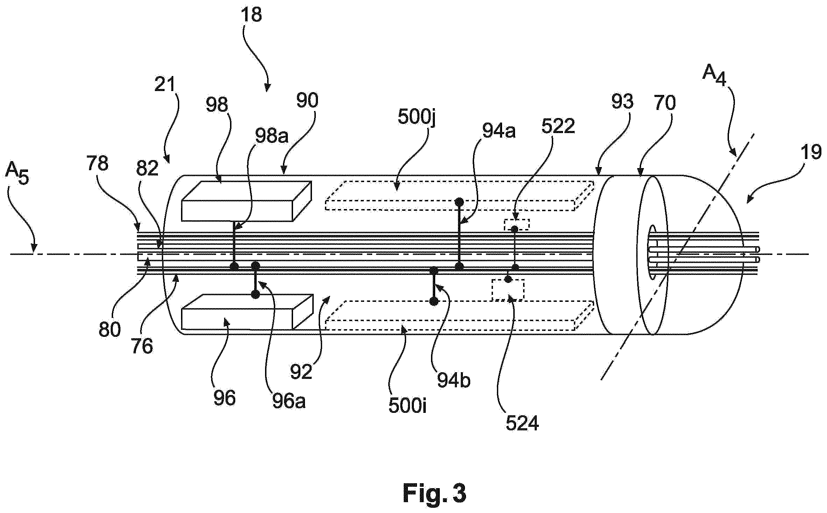

Jedem Gelenk 11, 13, 15, 17, 19, 21, 23 ist jeweils eine Bremse 60, 62, 64, 66, 68, 70, 72 zugeordnet. Dem Gelenk 11 ist die Bremse 60 zugeordnet, dem Gelenk 13 die Bremse 62, dem Gelenk 15 die Bremse 64, dem Gelenk 17 die Bremse 66, dem Gelenk 19 die Bremse 68, dem Gelenk 21 die Bremse 70 und dem Gelenk 23 die Bremse 72. Alle Bremsen 60 bis 72 sind als elektromagnetische Bremsen mit einem Permanentmagnet ausgebildet, derart, dass diese unbestromt in einen arretierten Zustand vorgespannt sind. Der Permanentmagnet ist derart ausgelegt, dass dieser das jeweilige Gelenk alleine arretieren kann und die Pose des Haltearms 1 gehalten wird. In dem nullten Armsegment 10 ist eine elektronische Steuereinheit 74 vorgesehen. Diese ist über ein Bussystem 76 (in Figur 2 nur im Armsegment 10 gezeigt; vgl. Fig. 3 und 4) mit allen Schnittstellen 500a bis 500n der Bedieneinheit 28 sowie mit allen Bremsen 60 bis 72 gekoppelt. Zur Energieversorgung der Bremsen 60 bis 72 sowie der Schnittstellen 500a bis 500n ist ferner eine Energieleitung 78 vorgesehen, die über die Schnittstelle 6 am proximalen Ende 2 des Haltearms 1 mit einer Energiequelle koppelbar ist.

Figur 3 zeigt exemplarisch das vierte Armsegment 18 in vergrößerter Darstellung teilweise aufgebrochen. Es soll erkannt werden, dass auch die anderen Armsegmente 10, 12, 14, 16, 20, 22 ebenso ausgebildet sein können.

Das Armsegment 18 weist einen Armsegmentkörper 90 auf (in den Fig. 1 und 2 nicht gezeigt; es soll verstanden werden, dass jedes Armsegment 10 bis 22 einen Armsegmentkörper hat), der gemäß Figur 3 im Wesentlichen stabförmig beziehungsweise zylindrisch ausgebildet ist. Im Inneren weist der Armsegmentkörper 90 einen Hohlraum 92 auf, in dem verschiedene Elemente, wie etwa die Bremse 70, angeordnet sind. Schematisch dargestellt in Figur 3 sind die Gelenke 19, 21 sowie die beiden Schwenkachsen A₄, A₅ der Gelenke 19, 21, die jeweils mit dem Haltearmsegment 18 zusammenwirken. Dem Haltearmsegment 18 ist das Gelenk 19 zugeordnet (vgl. obige Beschreibung zu den Fig. 1 und 2). Der Armsegmentkörper 90 weist eine äußere Oberfläche 93 auf, die im Wesentlichen zylindrisch ausgebildet ist. Der Armsegmentkörper 90 ist beispielsweise aus einem Metall wie insbesondere Aluminium oder Titan, einer Legierungen auf Basis von Aluminium oder Titan, oder einem Faserverbundwerkstoff, wie etwas GFK oder CFK, gebildet und vorzugsweise in Leichtbauweise ausgelegt.

Das Armsegment 18 weist gemäß Figur 3 die Schnittstellen 500i, 500j auf, welche Teil der Bedieneinheit 28 sind (vgl. Fig. 1 und 2). Die beiden Schnittstellen 500i, 500j sind im Wesentlichen gegenüberliegend bezogen auf die Achse A₅ angeordnet, sodass ein Bediener beim Ergreifen des Armsegments 18, wenn an den Schnittstellen 500i, 500j entsprechende Bedieneinrichtungen samt Schlüssel angeordnet sind, in Kontakt mit beiden Bedieneinrichtungen kommt.

Die beiden Schnittstellen 500i, 500j sind mittels Leitungen 94a, 94b mit dem Bussystem 76 gekoppelt. Über das Bussystem 76 sind die Schnittstellen 500i, 500j mit der elektronischen Steuereinheit 74 (vgl. Fig. 2) gekoppelt und über diese wiederum mit der Bremse 70, sodass die Bremse 70 durch die Bedieneinheit 28 freigegeben wird, wenn ein Bediener mit in den Schnittstellen 500i, 500j aufgenommenen Bedieneinrichtungen in Kontakt kommt.

Neben dem Bussystem 76 sind innerhalb des Armsegmentkörpers 90 ein Energieübertragungssystem 78 sowie ein Kabelkanal 80 und ein Arbeitskanal 82 angeordnet. Mittels des Energieübertragungssystems 78 sind die Schnittstellen 500i, 500j sowie die Bremse 70 an eine Energieversorgung angeschlossen.

Alternativ oder zusätzlich ist in jedem Armsegment ein Elektronikmodul 96 angeordnet, welches über eine Leitung 96a mit dem Bussystem 76 gekoppelt ist. In einem solchen Fall wirken die Schnittstellen 500i, 500j, welche über die Leitung 94a, 94b mit dem Datenbussystem 76 verbunden sind, nur mit dem Elektronikmodul 96 zusammen, welches den durch in den Schnittstellen 500i, 500j aufgenommenen Bedieneinrichtungen Signale in ein Stellsignal für die Bremse 70 umwandelt und dieses Stellsignal über das Bussystem 76 an die Bremse 70 sendet zum Freigeben des Gelenks 19. Ist in jedem Armsegment ein derartiges Elektronikmodul 96 angeordnet, ist ein im Wesentlichen modularer Aufbau des Haltearms 1 realisiert, und die einzelnen Armsegmente 10 bis 22 sind unabhängig von der elektronischen Steuereinheit 74, welche im proximalen Armsegment 10 angeordnet ist.

Der Kabelkanal 80 dient dazu, Kabel, die vom proximalen Ende 2 bis zum distalen Ende 4 verlaufen, um insbesondere die Schnittstelle 8 zu versorgen, führen zu können. Der Arbeitskanal 82 dient dazu, Schläuche oder Lichtleiter und dergleichen aufzunehmen, die je nach Art des Manipulator, der an der Schnittstelle 8 angeordnet ist, benötigt werden. Ist beispielsweise an der Schnittstelle 8 ein Endoskop angeordnet, wird vorzugsweise durch den Arbeitskanal 82 ein Lichtleiter geführt, der ein Bild, welches eine Endoskopkamera aufnimmt, übertragen kann. Der Arbeitskanal 82 dient demnach dazu, je nach Anwendungsgebiet entsprechende Übertragungsmittel aufzunehmen.

Weiterhin ist in dem Armsegment 18 ein Sensor 98 angeordnet. Vorzugsweise ist in jedem Armsegment 10 bis 22 ein Sensor angeordnet, und es soll verstanden werden, dass die Sensoren in den Armsegmenten 10, 12, 14, 16, 20 und 22 ebenso ausgebildet sein können wie der Sensor 98 im Armsegment 18. Der Sensor 98 ist vorzugsweise als Beschleunigungssensor ausgebildet. Indem in jedem Armsegment ein solcher Beschleunigungssensor vorgesehen ist, ist es möglich, zu jeder Zeit die Pose des Haltearms 1 zu bestimmen. Dazu ist der Sensor 98 über eine Leitung 98a mit dem Datenbussystem 76 gekoppelt, sodass die von dem Sensor 98 erfassten Daten an die elektronische Steuereinheit 74 übertragen werden, welche dann aus allen Sensordaten aus allen Armsegmenten die Pose des Haltearms 1 bestimmt. Ferner ist durch das Vorsehen eines derartigen Sensors 98 auch die absolute und relative Position eines an der Schnittstelle 8 angeordneten Endeffektors beziehungsweise Manipulators bestimmbar. Auch ist es möglich, wenn der Haltearm 1 an einem Operationstisch angeordnet ist, eine Bewegung dieses Operationstischs zu erkennen. Erfassen alle Sensoren in allen Armsegmenten eine Bewegung in dieselbe Richtung, ist dies ein Indikator dafür, dass der gesamte Haltearm 1 unter Beibehaltung seiner Pose bewegt wurde, beispielsweise dadurch, dass der Operationstisch oder eine Operationstischplatte relativ zu einer Säule der Operationstisches rotiert oder verschoben wurde. Auch eine solche Bewegung ist mittels der Sensoren 98 erfassbar. Ferner können äußere Impulse, wie etwa Stöße auf den Haltearm 1, erfasst werden.

In Figur 4 ist zunächst ein Abschnitt des Haltearms 1 gezeigt, allerdings nur exemplarisch. In dem Armsegment 20 sind zwei Schnittstellen 500k, 500l gegenüberliegend ausgebildet, in denen gemäß Figur 4 zwei Schlüssel 510k, 510l (insgesamt mit 510 bezeichnet) aufgenommen sind. Die Schlüssel 510 sind jeweils an einer Bedieneinrichtung 520k, 520l (insgesamt mit 520 bezeichnet) angeordnet. Die Bedieneinrichtungen 520 dienen dazu, einen Kontakt einer Bedienperson aufzunehmen und in ein Signal zu wandeln, welches von der Steuereinheit 74 in ein Stellsignal für die Bremsen gewandelt wird. Die Schlüssel 510k, 510l haben eine Identität, die in den Schlüsseln codiert ist. Da die Schlüssel mit den Bedieneinrichtungen 520k, 520l verbunden sind, codieren die Schlüssel 510k, 510l auch die Identität der Bedieneinrichtungen 520k, 520l. In dem Haltearmsegment 20 sind ferner zwei Erfassungsmittel 522, 524 vorgesehen, mittels derer die Identität der Schlüssel 510k, 510l erfassbar ist. Die Erfassungsmittel 522, 524 sind mit dem Datenbussystem 76 gekoppelt, wie in Figur 3 gezeigt.

Gemäß Figur 5 weist die Bedieneinrichtung 520 im Wesentlichen einen flächigen Grundkörper 526 auf, der eine Eingabeeinrichtung 528 zum Eingeben von Steuersignalen durch einen Bediener aufweist. Die Eingabeeinrichtung 528 ist bevorzugt als berührungsempfindliche Oberfläche ausgebildet, die im Inneren mit einem entsprechenden Mikrocontroller verbunden ist, der über einen Anschluss 530 entsprechend Signale an die Schnittstelle 500, in der ein entsprechender zweiter Anschluss 532 vorgesehen ist, übertragen kann. Über den Anschluss 532 werden die erfassten Signale von der Eingabeeinrichtung 528 über eine Verbindung 534 an das Bussystem 76 übergeben.

Der Schlüssel 510 weist gemäß diesem Ausführungsbeispiel einen im Wesentlichen zapfenförmigen Körper 536 auf, der an seiner Außenseite gestuft ist. Neben dem Anschluss 530 weist der Schlüssel 510 elektrische Kontakte 538, 539, 540 auf, die in Kontakt mit entsprechenden elektrischen Kontakten 542, 543, 544 der Schnittstelle 500 kommen können. Über diese elektrischen Kontakte 538, 539, 540, 542, 543, 544 kann ein Chip im Inneren des Schlüssels 510 ausgelesen werden, oder das Erfassungsmittel 522 erfasst aufgrund der Anzahl und/oder Art der geschlossenen Kontakte die Identität des Schlüssels 510. Indem der Schlüssel 510 den Zapfen 536 aufweist, ist gleichzeitig eine mechanische Kopplung zwischen der Bedieneinrichtung 520 und dem Armsegment 20 erreicht. Zusätzlich kann auch eine magnetische Kopplung und/oder eine Rastverbindung vorgesehen sein.

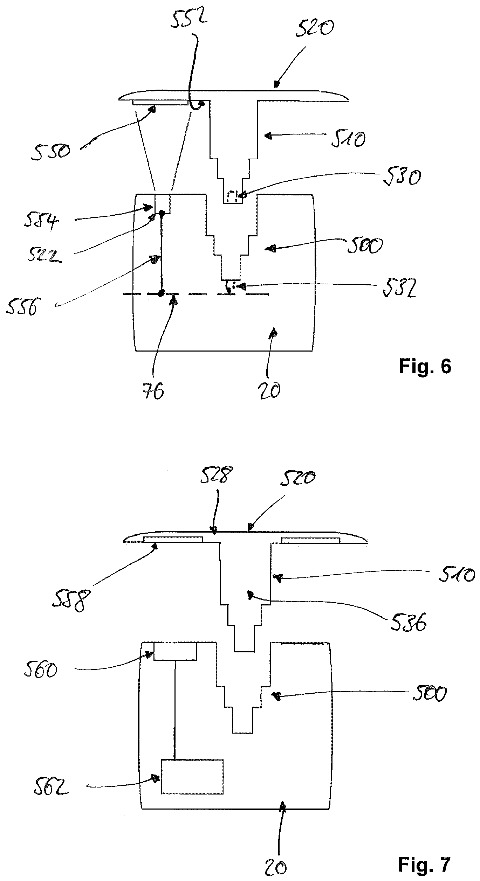

Die Figuren 6 und 7 zeigen weitere Ausführungsformen des Schlüssels 510. Gleiche und ähnliche Elemente sind mit gleichen Bezugszeichen versehen, und insofern wird vollumfänglich auf die obige Beschreibung zu den Figuren 4 und 5 verwiesen.

In Figur 6 ist wiederum das Armsegment 20 dargestellt, in dem die Schnittstelle 500 ausgebildet ist. An dem Schlüssel 510 ist wiederum ein Kontakt 530 vorgesehen, der mit dem Kontakt 532 eine Signalübermittlung von der Bedieneinrichtung 520 zum Bussystem 76 ermöglicht. Gemäß diesem Ausführungsbeispiel weist der Schlüssel 510 eine optische Kodierung in Form eines QR-Codes 550 auf. Der QR-Code ist auf einer Unterseite 552 der Bedieneinrichtung 520 angeordnet und durch einen entsprechenden Sensor 554, der in dem Haltearmsegment 20 vorgesehen ist, erfassbar. Der Sensor 554 bildet so das Erfassungsmittel 522. Der Sensor 554 ist mittels einer Leitung 556 mit dem Bussystem 76 gekoppelt, um die erfasste Identität des Schlüssels 510 über das Bussystem 76 an die Steuereinheit 74 (siehe Fig. 1 und 2) zu übermitteln. Aufgrund des erfassten Barcodes 550 steuert die Steuereinheit dann die Freigabeeinrichtung. Über den Barcode 550 können ferner weitere Informationen und Daten über den Sensor 554 abgegriffen oder an das Datenbussystem 76 übertragen werden. Solche Informationen können zusätzliche Beschränkungen oder Freiheitsgrade für den Haltearm 1 umfassen.

In Figur 7 ist schematisch eine weitere bevorzugte Variante des Schlüssels 510 dargestellt. Wiederum sind gleiche und ähnliche Elemente mit gleichen Bezugszeichen versehen und insofern wird vollumfänglich auf die obige Beschreibung zu den Figuren 4 bis 6 Bezug genommen. Auch wenn in Figur 7 das Bussystem 76 und die Kontakte 530, 532 nicht explizit gezeigt sind, soll verstanden werden, dass diese bei der Ausführungsform gemäß Figur 7 dennoch vorhanden sind.

Der Schlüssel 510 gemäß Figur 7 weist an der Bedieneinrichtung 520 einen RFID-Transponder 558 auf, und an dem Armsegment ist ein entsprechendes RFID-Lesegerät 560 vorgesehen. Das Lesegerät 560 ist mit einer Auswerteeinheit 562 gekoppelt, die wiederum mit dem Bussystem 76 (in Fig. 7 nicht gezeigt) verbunden ist. Über den RFID-Transponder 558 ist die Identität des Schlüssels 510 auslesbar, wenn der RFID-Transponder 558 im Bereich der Leseeinheit 560 ist. Dies ist der Fall, wenn der Zapfen 536 vollständig in der Schnittstelle 500 aufgenommen ist und so eine mechanische Kopplung zwischen der Bedieneinrichtung 520 und dem Haltearmsegment 20 erreicht ist. Mittels der Auswerteeinheit 562 können zudem über die Leseeinheit 560 Informationen und Daten an die Bedieneinrichtung 528 übergeben werden. Dies ist besonders vorteilhaft, wenn diese beispielsweise einen Bildschirm oder dergleichen aufweist, auf dem Daten, wie Patientendaten oder dergleichen angezeigt werden.

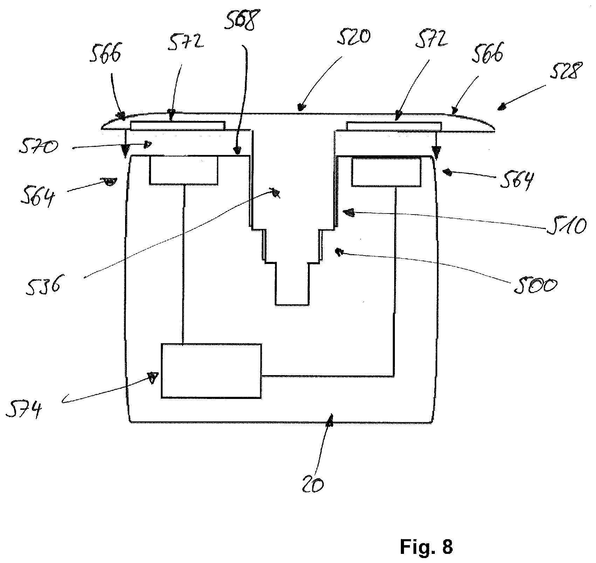

Ausweilich Figur 8 weist das Armsegment 20 zusätzlich oder alternativ ferner zwei kapazitive Sensoren 564 auf. Zwischen auskragenden Abschnitten 566 der Bedieneinrichtung 520 und einer Oberfläche 558 des Armsegments 20 ist ein Spalt 570 vorgesehen. Die kapazitiven Sensoren 564 sind so ausgebildet, dass sie kleinste Abstandsänderungen zwischen einem in den auskragenden Abschnitten 566 vorgesehenen Sensorelement 572 und den Sensoren 564 erfassen. Der Zapfen des Schlüssels 510 kann leicht elastisch sein, oder die auskragenden Abschnitte 566 sind leicht elastisch, sodass bei Kontakt von einem Bediener und der Bedieneinrichtung 520 der Abstand zwischen den Sensorelementen 572 und den Sensoren 564 verändert wird, wodurch die Sensoren 564 ein Signal an eine Auswerteeinheit 574 bereitstellen, welche dieses Signal wiederum über das Datenbussystem 76 an die Steuereinheit 74 weitergibt. Dies ist eine Alternative zu einer berührungsempfindlichen Oberfläche 528, wie sie mit Bezug auf Figur 5 beschrieben wurde. Der Vorteil einer solchen Ausgestaltung ist, dass die Bedieneinrichtung 520 vollständig sterilisierbar, insbesondere autoklavierbar ist, da diese keine empfindlichen elektronischen Bauteile aufweist.

Sind an den Schnittstellen 500 (vgl. Fig. 1) jeweils entsprechende Bedieneinrichtungen 520 vorgesehen, kommt der Bediener beim Ergreifen eines Armsegments 10, 12, 14, 16, 18, 20, 22 (vgl. Fig. 1) in Kontakt mit jeweils beiden gegenüberliegenden Bedieneinrichtungen 520, und nur bei Kontakt mit beiden Eingabeeinrichtungen 528, die Kontaktmittel bilden, einer Bedieneinrichtung 520 wird das zugeordnete Gelenk freigegeben. Das heißt, beim Ergreifen des ersten Armsegments 12 und gleichzeitigem Inkontaktkommen mit den beiden gegenüberliegenden Eingabeeinrichtungen 528 wird durch die Bedieneinheit 28 das erste Gelenk 13 freigegeben. Dadurch ist es möglich, dass der Bediener den Haltearm 1 beziehungsweise die Armsegmente 12 bis 22 um die Achse A₁ verschwenken kann. Bei Loslassen eines der beiden oder beider Eingabeeinrichtungen 528 wird das Gelenk 13 wieder arretiert, und ein Verschwenken um die Achse A₁ ist nicht mehr möglich. Auch bei einer unbeabsichtigten Berührung nur eines der beiden Eingabeeinrichtungen 528, beispielsweise mit einem Arm oder Ellenbogen des Bedieners, wird das Gelenk 13 nicht freigegeben, und der Haltearm 1 bleibt im arretierten Zustand und hält seine Pose. Entsprechendes gilt für die weiteren Armsegmente. Die Bedieneinheit 28 kann dazu einen Controller oder Mikroprozessor aufweisen, der dazu eingerichtet ist, einen Kontakt zwischen Eingabeeinrichtungen 528 zu erfassen und in elektrische Signale zu übertragen.

Gemäß Figur 9 ist ein Haltearm 1 gemäß einem zweiten Ausführungsbeispiel der Erfindung gezeigt. Der Haltearm ist im Wesentlichen identisch zu dem aus den Figuren 1 und 2 gebildet und weist sieben Armsegmente 10, 12, 14, 16, 18, 20, 22 auf. Im Unterschied zu den vorherigen Ausführungsbeispielen weist der Haltearm 1 gemäß Figur 9 nur eine Schnittstelle 500 auf, die im Armsegment 20 vorgesehen ist. An den weiteren Armsegmenten 10, 12, 14, 16, 18, 22 können beispielsweise Kontaktflächen wie in DE 10 2014 016 824 offenbart ausgebildet sein. Alternativ können die Armsegmente 10, 12, 14, 16, 18, 20, 22 auf andere Art und Weise zueinander bewegt werden, beispielsweise durch aktive Motoren in den Gelenken, sodass der Haltearm 1 gemäß dem in Figur 9 gezeigten Ausführungsbeispiel als aktiver Haltearm ausgebildet ist. Die Schnittstelle 500 dient zur Aufnahme eines Schlüssels, wobei an der Schnittstelle 500 ein Erfassungsmittel 528 wie beispielsweise in den Figuren 5 bis 7 offenbart ausgebildet ist. Der Haltearm 1 gemäß Figur 9 weist ferner eine Steuereinheit und eine Freigabeeinrichtung auf, wobei die Steuereinheit dazu eingerichtet ist, die Freigabeeinrichtung in Abhängigkeit von der Identität des aufgenommenen Schlüssels zu steuern.

Gemäß diesem Ausführungsbeispiel kann ein Operateur einen passenden Schlüssel 510 auswählen und diesen in der Schnittstelle 500 anordnen. Aufgrund der erfassten Identität des Schlüssels 510 wird dann mittels der Steuereinheit 76 die Freigabeeinrichtung gesteuert.

## Patentansprüche

1. Haltearm (1) für medizinische Zwecke, insbesondere zum Halten eines chirurgischen mechatronischen Assistenzsystems und/oder eines chirurgischen Instruments, mit :
einem proximalen Ende (2) zum Befestigen des Haltearms (1) an einer Basis und einem distalen Ende (4) zum Aufnehmen eines chirurgischen mechatronischen Assistenzsystems und/oder chirurgischen Instruments;
wenigstens einem ersten und einem zweiten Armsegment (12, 14), wobei das erste Armsegment (12) mit einem ersten Gelenk (13) und das zweite Armsegment (14) mit einem zweiten Gelenk (15) verbunden ist;
eine Freigabeeinrichtung, wobei jedes Gelenk (13, 15) mittels der Freigabeeinrichtung freigebbar und arretierbar ist;
einer Bedieneinheit (28) zum Verbringen des Haltearms (1) in eine gewünschte Pose;
einer Steuereinheit (74), die mit der Bedieneinheit (28) und der Freigabeeinrichtung zum Übertragen von Signalen von der Bedieneinheit (28) an die Freigabeeinrichtung gekoppelt ist,
**gekennzeichnet durch** eine Schnittstelle (500) zur Aufnahme eines Schlüssels (510), und
durch ein Erfassungsmittel (522, 524) zum Erfassen einer Identität des Schlüssels (510),
wobei die Steuereinheit (74) dazu eingerichtet ist, die Freigabeeinrichtung in Abhängigkeit von der Identität des aufgenommenen Schlüssels (510) zu steuern.

2. Haltearm nach Anspruch 1, wobei der Schlüssel (510) als Daten-Schlüssel oder als Software-Schlüssel ausgebildet ist.

3. Haltearm nach Anspruch 1 oder 2, wobei die Steuereinheit (76) dazu eingerichtet ist, die Freigabeeinrichtung so in Abhängigkeit von der Identität des aufgenommenen Schlüssels (510) zu steuern, dass bei Erfassen einer ersten Identität eines ersten Schlüssels (510) eine erste Mehrzahl an Beschränkungen für den Haltearm (1) vorgesehen ist, und dass bei Erfassen einer zweiten Identität eines zweiten Schlüssels eine zweite Mehrzahl an Beschränkungen für den Haltearm (1) vorgesehen ist.

4. Haltearm nach einem der vorstehenden Ansprüche, wobei das Erfassungsmittel (522, 524) ein optisches Erfassungsmittel zum optischen Erfassen der Identität des Schlüssels (510) aufweist.

5. Haltearm nach einem der vorstehenden Ansprüche, wobei das Erfassungsmittel (522, 524) einen Empfänger zum Empfangen elektromagnetischer Wellen aufweist, wobei die elektromagnetischen Wellen die Identität des Schlüssels (510) repräsentieren.

6. Haltearm nach einem der vorstehenden Ansprüche, wobei das Erfassungsmittel (522, 524) eine elektronische Schnittstelle zum Empfangen eines Identifikationssignals des Schlüssels (510) aufweist.

7. Haltearm nach einem der vorstehenden Ansprüche, wobei das Erfassungsmittel (522, 524) ein elektromechanisches Schloss zur Aufnahme des Schlüssels (510) aufweist.

8. Haltearm nach einem der vorstehenden Ansprüche, wobei an dem distalen Ende (4) ein chirurgisches mechatronisches Assistenzsystem aufgenommen ist und die Bedieneinrichtung (520) und/oder die Schnittstelle (500) an dem Assistenzsystem angeordnet ist.

9. Bedieneinrichtung (520) für einen Haltearm (1) nach Anspruch 1, aufweisend einen Schlüssel (510) für die Schnittstelle (500) zur Aufnahme eines Schlüssels (510).

10. Bedieneinrichtung (520) nach Anspruch 9, ferner aufweisend einen Grundköper (526),
eine Eingabeeinrichtung (528), zum Eingeben eines Steuersignals durch einen Bediener,
eine Schnittstelle zum Übertragen des Steuersignals an den Haltearm (1),
wobei der Schlüssel eine Identität der Bedieneinrichtung (520) angibt.

11. Bedieneinrichtung nach Anspruch 9 oder 10, wobei der Schlüssel (500) eine optische Kodierung (550) aufweist, wobei die optische Kodierung (550) vorzugsweise eine farbliche Kodierung oder eine strukturelle Kodierung aufweist.

12. Bedieneinrichtung nach einem der Ansprüche 9 bis 11, wobei der Schlüssel (510) einen Sender (558) zum Senden elektromagnetischer Wellen aufweist.

13. Bedieneinrichtung nach einem der Ansprüche 9 bis 12, wobei dar Schlüssel (510) eine elektronische Schnittstelle (530) zum Senden eines Identifikationssignals aufweist.

14. Bedieneinrichtung nach einem der Ansprüche 9 bis 13, aufweisend Anzeigemittel, vorzugsweise Beleuchtungsmittel, zum Anzeigen eines Zustands der Bedieneinrichtung und/oder des Haltearms und/oder eines an dem Haltearm angeschlossenen mechatronischen Assistenzsystems und/oder chirurgischen Instruments.

15. Bedieneinrichtung nach einem der Ansprüche 9 bis 14, aufweisend einen Bildschirm zum Anzeigen von Daten und/oder eines Patientenmodells.

16. Bedieneinrichtung nach einem der Ansprüche 9 bis 15, aufweisend eine elektronische Spule für eine chirurgische Navigation.

## Claims

1. A mounting arm (1) for medical purposes, in particular for mounting a surgical mechatronic assistance system and/or a surgical instrument, having:
a proximal end (2) for attaching the mounting arms (1) to a base and a distal end (4) for receiving a surgical mechatronic assistance system and/or surgical instrument;
at least a first and a second arm segment (12, 14), wherein the first arm segment (12) is connected to a first joint (13) and the second arm segment (14) is connected to a second joint (15);
a release device, wherein each joint (13, 15) can be released and locked by means of the release device;
an operating unit (28) for bringing the mounting arm (1) into a desired pose;
a control unit (74), which is coupled to the operating unit (28) and to the release device for transmitting signals from the operating unit (28) to the release device,
**characterized by** an interface (500) for receiving a key (510), and
by a detection means (522, 524) for detecting an identity of the key (510),
wherein the control unit (74) is set up to control the release device as a function of the identity of the received key (510).

2. The mounting arm according to claim 1, wherein the key (510) is implemented as a data key or as a software key.

3. The holding arm according to claim 1 or 2, wherein the control unit (76) is set up to control the release device as a function of the identity of the received key (510) such that upon detecting a first identity of a first key (510), a first plurality of restrictions is provided for the mounting arm (1), and in that upon detection of a second identity of a second key, a second plurality of restrictions is provided for the mounting arm (1).

4. The mounting arm according to one of the preceding claims, wherein the detection means (522, 524) comprises optical detection means for optically detecting the identity of the key (510).

5. The mounting arm according to one of the preceding claims, wherein the detection means (522, 524) comprises a receiver for receiving electromagnetic waves, wherein the electromagnetic waves represent the identity of the key (510).

6. The mounting arm according to one of the preceding claims, wherein the detection means (522, 524) comprises an electronic interface for receiving an identification signal of the key (510).

7. The mounting arm according to one of the preceding claims, wherein the detection means (522, 524) comprises an electromechanical lock for receiving the key (510).

8. The mounting arm according to one of the preceding claims, wherein a surgical mechatronic assistance system is received at the distal end (4) and the operating device (520) and/or the interface (500) is disposed on the assistance system.

9. An operating device (520) for a mounting arm (1) according to claim 1, comprising a key (510) for the interface (500) for receiving a key (510).

10. The operating device (520) according to claim 9, further comprising a base body (526),
an input device (528) for inputting a control signal by an operator,
an interface for transmitting the control signal to the mounting arm (1),
wherein the key indicates an identity of the operating device (520).

11. The operating device according to claim 9 or 10, wherein the key (500) comprises an optical coding (550), wherein the optical coding (550) preferably comprises a color coding or a structural coding.

12. The operating device according to one of claims 9 to 11, wherein the key (510) comprises a transmitter (558) for transmitting electromagnetic waves.

13. The operating device according to one of claims 9 to 12, wherein the key (510) comprises an electronic interface (530) for transmitting an identification signal.

14. The operating device according to one of claims 9 to 13, comprising indicating means, preferably illumination means, for displaying a state of the operating device and/or the mounting arm and/or a mechatronic assistance system and/or surgical instrument connected to the holding arm.

15. The operating device according to one of claims 9 to 14, comprising a screen for displaying data and/or a patient model.

16. The operating device according to one of claims 9 to 15, comprising an electronic coil for a surgical navigation.

## Revendications

1. Bras (1) de retenue à des fins médicales, notamment pour retenir un système d'assistance chirurgicale mécatronique et/ou un instrument chirurgical, comprenant
une extrémité (2) proximale de fixation du bras (1) de retenue à une base et une extrémité (4) distale de réception d'un système d'assistance chirurgical mécatronique et/ou d'un instrument chirurgical ;
au moins un premier et un deuxième segments (12, 14) du bras, le premier segment (12) du bras étant relié à une première articulation (13) et le deuxième segment (14) du bras a une deuxième articulation (15) ;
un dispositif de libération, chaque articulation (13, 15) pouvant être libérée et bloquée au moyen du dispositif de libération ;
une unité (28) de service pour mettre le bras (1) de retenue dans une pose souhaitée ;
une unité (74) de commande, qui est couplée à l'unité (28) de service et au dispositif de libération, pour la transmission de signaux de l'unité (28) de service au dispositif de libération,
**caractérisé par** une interface (500) de réception d'une clé (510) et
par un moyen (522, 524) de détection pour la détection d'une identité de la clé (510),
dans lequel l'unité (74) de commande est conçue pour commander le dispositif de libération en fonction de l'identité de la clé (510) reçue.

2. Bras de retenue suivant la revendication 1, dans lequel la clé (510) est constituée sous la forme d'une clé de données ou sous la forme d'une clé de logiciel.

3. Bras de retenue suivant la revendication 1 ou 2, dans lequel l'unité (76) de commande est conçue pour commander le dispositif de libération en fonction de l'identité de la clé (510) reçue de manière à ce que, à la détection d'une première identité d'une première clé (510), soit prévue une première pluralité de restrictions pour le bras (1) de retenue et de manière à ce que, à la détection d'une deuxième identité d'une deuxième clé, soit prévue une deuxième pluralité de restrictions pour le bras (1) de retenue.

4. Bras de retenue suivant l'une des revendications précédentes, dans lequel le moyen (522, 524) de détection a un moyen de détection optique pour la détection optique de l'identité de la clé (510).

5. Bras de retenue suivant l'une des revendications précédentes, dans lequel le moyen (522, 524) de détection a un récepteur pour la réception d'ondes électromagnétiques, les ondes électromagnétiques représentant l'identité de la clé (510).

6. Bras de retenue suivant l'une des revendications précédentes, dans lequel le moyen (522, 524) de détection a une interface électronique de réception d'un signal d'identification de la clé (510).

7. Bras de retenue suivant l'une des revendications précédentes, dans lequel le moyen (522, 524) de détection a une serrure électromécanique de réception de la clé (510).

8. Bras de retenue suivant l'une des revendications précédentes, dans lequel un système d'assistance chirurgicale mécatronique est reçu à l'extrémité (4) distale et le dispositif (520) de service et/ou l'interface (500) est monté sur le système d'assistance.

9. Dispositif (520) de service pour un bras (1) de retenue suivant la revendication 1, comportant une clé (510) pour l'interface (500) de réception d'une clé (510).

10. Dispositif (520) de service suivant la revendication 9, comportant, en outre, une embase (526),
un dispositif (528) d'entrée pour l'entrée d'un signal de commande par un opérateur,
une interface de transmission du signal de commande au bras (1) de retenue,
la clé indiquant une identité du dispositif (520) de service.

11. Dispositif (520) de service suivant la revendication 9 ou 10, dans lequel la clé (500) a un code (550) optique, le code (550) optique ayant, de préférence, un codage de couleur ou un codage de structure.

12. Dispositif (520) de service suivant l'une des revendications 9 à 11, dans lequel la clé (510) a un émetteur (558) d'émission d'ondes électromagnétiques.

13. Dispositif (520) de service suivant l'une des revendications 9 à 12, dans lequel la clé (510) a une interface (530) électronique d'émission d'un signal d'identification.

14. Dispositif (520) de service suivant l'une des revendications 9 à 13, ayant un moyen d'indication, de préférence un moyen d'éclairage, pour indiquer un état du dispositif de service et/ou du bras de retenue et/ou d'un système d'assistance mécatronique et/ou d'un instrument chirurgical raccordé au bras de retenue.

15. Dispositif (520) de service suivant l'une des revendications 9 à 14, comportant un écran d'affichage de données et/ou d'un modèle de patient.

16. Dispositif (520) de service suivant l'une des revendications 9 à 15, comportant une bobine électronique pour une navigation chirurgicale.
